(19) Europäisches Patentamt
European Patent Office
Office européen des brevets

(11) EP 2 566 331 B1

(12) EUROPEAN PATENT SPECIFICATION

(45) Date of publication and mention of the grant of the patent:
**03.09.2014 Bulletin 2014/36**

(21) Application number: **11778348.0**

(22) Date of filing: **05.05.2011**

(51) Int Cl.:
***A61K 9/00*** (2006.01) ***A61K 31/506*** (2006.01)
***A61K 47/40*** (2006.01) ***A61K 47/48*** (2006.01)

(86) International application number:
**PCT/US2011/035363**

(87) International publication number:
**WO 2011/140343 (10.11.2011 Gazette 2011/45)**

(54) **PHARMACEUTICAL COMPOSITIONS AND METHODS OF MAKING SAME**

PHARMAZEUTISCHE ZUSAMMENSETZUNGEN UND VERFAHREN ZU IHRER HERSTELLUNG

COMPOSITIONS PHARMACEUTIQUES ET PROCÉDÉS POUR LES FABRIQUER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.05.2010 US 331715 P**

(43) Date of publication of application:
**13.03.2013 Bulletin 2013/11**

(73) Proprietor: **Glaxo Wellcome Manufacturing Pte Ltd**
**Jurong 628413 (SG)**

(72) Inventor: **GUPTA, Manish K.**
**Collegeville**
**Pennsylvania 19426 (US)**

(74) Representative: **Learoyd, Stephanie Anne**
**GlaxoSmithKline**
**Global Patents (CN925.1)**
**980 Great West Road**
**Brentford, Middlesex TW8 9GS (GB)**

(56) References cited:
**EP-B1- 1 968 594** **WO-A1-2011/039648**
**US-A1- 2007 244 166** **US-A1- 2009 304 694**

- **CARRIER ET AL.: 'The Utility of Cyclodextrins for Enhancing Oral Bioavailability.' JOURNAL OF CONTROLLED RELEASE vol. 123, 2007, pages 78 - 99**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 566 331 B1

## Description

FIELD OF THE INVENTION

This invention relates to pharmaceutical compositions and methods of making the same, particularly pharmaceutical formulations suitable for ocular administration.

BACKGROUND

Pazopanib is a highly bio-available, multi- tyrosine kinase inhibitor of vascular endothelial growth factor receptor (VEGFR)-1, -2, -3, platelet-derived factor receptor (PDGFR) -$\alpha$, -$\beta$, cytokine receptor (cKit), interleukin-2 receptor inducible T-cell kinase (Itk), leukocyte-specific protein tyrosine kinase (Lck), and transmembrane glycoprotein receptor tyrosine kinase (c-Fms). WO 2007/064752 describes the use of pazopanib to treat age-related macular degeneration. It is desirable to provide a stable eye-drop formulation of pazopanib in which the pazopanib is solubilized in the formulation. Pazopanib is a poorly water-soluble drug, having a solubility in phosphate buffer of approximately 0.000006 mg/mL (1.37 x $10^{-8}$ mol/L) at pH 5.0 and a temperature of approximately 25°C.

Cyclodextrins are used in drug formulations as solubility enhancers because of their ability to form water-soluble inclusion complexes with otherwise poorly water-soluble drugs. The fundamental property that describes the strength of interaction between a drug and a cyclodextrin is the binding constant (or stability constant) K. The cyclodextrin utility number ($U_{CD}$) is a dimensionless number that can be used to assess the feasibility of the use of cyclodextrins in dosage forms. The $U_{CD}$ allows the formulator to determine if the use of cyclodextrins in the formulation of poorly water-soluble drugs has the potential to provide a significant solubilization advantage. $U_{CD}$ is calculated using the following equation:

$$U_{CD} = (KS_o/1+KS_o)(m_{CD}/m_D)(\ MW_D/MW_{CD})$$

where:

- $m_D$ is dose of drug;
- $m_{CD}$ is dose of cyclodextrin;
- $MW_D$ is molecular weight drug;
- $MW_{CD}$ is molecular weight cyclodextrin; and
- $K_o$ is binding constant.

When the dimensionless number $U_{CD}$ is greater than or equal to 1, solubilization is adequately provided by the complexation of cyclodextrins with the drug. When the dimensionless number is less than 1, the complexation alone is not enough for complete solubilization. For ophthalmic formulations, the workable amount of the cyclodextrin, $m_{CD}$, can depend upon the desired tonicity of the solution. *See*, V.M. Rao & V.J. Stella, When Can Cyclodextrins Be Considered for Solubilization Purposes?, J. Pharm. Sci., Vol. 92, No. 5 (2003).

A poster entitled "A Multi-Targeted Receptor Tyrosine Kinase Inhibitor for the Treatment of Neovascular AMD: Preclinical Support of Clinical Development of Pazopanib Eye Drops" presented on May 6, 2009 at the annual meeting of the Association for Research in Vision and Ophthalmology (ARVO) ("the ARVO poster") reported an eye drop formulation for use in ocular toxicity studies of dogs and rabbits. The poster reported that the formulation included 7% modified cyclodextrin, sodium phosphate, sodium chloride and 0.01% benzalkonium chloride (with a note that benzalkonium chloride is not in the current clinical formulation) at a pH of 5. The poster reported 2 and 5 mg/ml concentrations of pazopanib.

In a preclinical toxicity study, it is important that the formulation does not change the toxic effect of the active ingredient (either mask or enhance it). It is very important because in many cases the final clinical formulation may be quite different in composition from the dosage forms used for safety assessment in animals. For example, the final clinical formulation will need to exhibit stability over a length of time that allows it to be manufactured, inventoried, delivered to the pharmacy, dispensed to the patient, and administered by the patient for the entire course of treatment for the given prescription. This time period can be as short as a month or two or as long as six months to a year or more. In contrast, the formulation used for a preclinical toxicity study does not have to exhibit long-term stability, but instead can be prepared within a few days of dosing.

With a few assumptions, a $U_{CD}$ number can be calculated based on the toxicity study formulation proposed in the ARVO poster. Pazopanib has a molecular weight of 437.5 and a modified cyclodextrin such as Captisol® ($\beta$-cyclodextrin sulfobutylether) has a molecular weight of 2,200. A value of binding constant of pazopanib, K, was determined to be approximately 10,000. As noted above, the solubility of pazopanib in phosphate buffer at a pH 5 and a temperature

of approximately 25°C is 0.000006 mg/mL, which corresponds to 1.37 x $10^{-8}$ mol/L. For a 2 mg/mL formulation of pazopanib using 7 % w/w Captisol® (sulfobutylether-cyclodextrin), the $U_{CD}$ value would be 0.001 at pH 5. For a 5 mg/mL formulation of pazopanib using 7 % w/w Captisol® (sulfobutylether-cyclodextrin), the $U_{CD}$ value would be 0.0004 at pH 5. As described above with reference to the Rao article, the $U_{CD}$ allows the formulator to determine if the use of cyclodextrins in the formulation of poorly water-soluble drugs has the potential to provide a significant solubilization advantage. When the dimensionless number is less than 1, the complexation alone is not enough for complete solubilization. In view of the very low $U_{CD}$ values for these pre-clinical toxicity study formulations, it would not be expected that formulations such as these would exhibit the stability necessary for use as clinical trial formulations.

It is desirable to provide a stable eye-drop formulation in which an amount of an acid addition salt of pazopanib equivalent to 10 mg/mL pazopanib free base is solubilized in the formulation.

## SUMMARY OF THE INVENTION

In one aspect of the present invention, a pharmaceutical composition includes about 10 mg pazopanib/mL of the composition, about 2.0 to about 13.0 % w/w of a modified cyclodextrin, said modified cyclodextrin being selected such that the modified cyclodextrin results in the $pK_a$ of pazopanib with said modified cyclodextrin in water being lower than the $pK_a$ of pazopanib alone in water, a pH adjusting agent as needed to provide a pH of 3.5 to 5.7, a tonicity adjusting agent as needed to provide an osmolality of 200 to 400 mOsm, and water. The composition is stable for at least 2 months.

In another aspect of the present invention, a pharmaceutical composition includes about 10 mg pazopanib/mL of the composition, about 2.0 to about 13.0 % w/w of a modified cyclodextrin, a pH adjusting agent as needed to provide a pH of 3.5 to 5.7, a tonicity adjusting agent as needed to provide an osmolality of 200 to 400 mOsm, and water. The composition is suitable for administration to the eye of a human and has a $U_{CD}$ value in the range of 0.0002 to 0.6 at a temperature of approximately 25°C. The composition is stable for at least 2 months.

In still another aspect of the present invention, a pharmaceutical composition includes about 10 mg pazopanib/mL of the composition, about 2.0 to about 13.0 % w/w of a modified cyclodextrin, a pH adjusting agent as needed to provide a pH of 3.5 to 5.7, a tonicity adjusting agent as needed to provide an osmolality of 200 to 400 mOsm, and water, where the composition is a super-saturated aqueous solution of pazopanib. The composition is stable for at least 2 months.

In yet another aspect of the present invention, a pharmaceutical composition includes about 10 mg pazopanib/mL of the composition, about 2.0 to about 13.0 % w/w of a modified cyclodextrin, a pH adjusting agent as needed to provide a pH of 3.5 to 5.7, a tonicity adjusting agent as needed to provide an osmolality of 200 to 400 mOsm, and water.

In another aspect of the present invention, a method of preparation of a super-saturated composition includes forming an aqueous solution of an acid addition salt of pazopanib and a modified cyclodextrin, and adjusting the pH of said solution to between about 4 to about 5 to obtain a super-saturated solution of pazopanib that is stable for at least 2 months.

## DETAILED DESCRIPTION OF THE INVENTION

As used herein, "super-saturated solution" means a solution containing more solute than would a saturated solution under given conditions of temperature and pressure.

As used herein, "stable" means the composition is physically stable according to United States Pharmacopeia (USP) <789> "Particulate Matter in Ophthalmic Solutions" when the composition is packaged in a blow-fill sealed single-use container that is overwrapped using blank flow wrap aluminum foil and the temperature of the packaged formulation is maintained at 5°C.

In one aspect of the present invention, a pharmaceutical composition includes about 10 mg pazopanib/mL of the composition, about 2.0 to about 13.0 % w/w of a modified cyclodextrin, said modified cyclodextrin being selected such that the modified cyclodextrin results in the $pK_a$ of pazopanib with said modified cyclodextrin in water being lower than the $pK_a$ of pazopanib alone in water, a pH adjusting agent as needed to provide a pH of 3.5 to 5.7, a tonicity adjusting agent as needed to provide an osmolality of 200 to 400 mOsm, and water. The composition is stable for at least 2 months.

In some embodiments according to this aspect of the present invention, the modified cyclodextrin is selected such that the modified cyclodextrin results in the $pK_a$ of pazopanib with said modified cyclodextrin in water being at least 0.4 lower than the $pK_a$ of pazopanib alone in water. In other embodiments according to this aspect of the present invention, the modified cyclodextrin is selected such that the modified cyclodextrin results in the $pK_a$ of pazopanib with a given amount of modified cyclodextrin in water being at least 0.8 lower than the $pK_a$ of pazopanib in water.

The $pK_a$ of pazopanib in water with a given amount of modified cyclodextrin can be measured by potentiometry as described in Example 7. The $pK_a$ of pazopanib in a 10 mg /mL water solution can be determined theoretically using ACD predictive software as will be understood by those skilled in the art.

In another aspect of the present invention, a pharmaceutical composition includes about 10 mg pazopanib/mL

of the composition, about 2.0 to about 13.0 % w/w of a modified cyclodextrin, a pH adjusting agent as needed to provide a pH of 3.5 to 5.7, a tonicity adjusting agent as needed to provide an osmolality of 200 to 400 mOsm, and water. The composition is suitable for administration to the eye of a human and has a $U_{CD}$ value in the range of 0.0002 to 0.6 at a temperature of approximately 25°C. The composition is stable for at least 2 months.

In still another aspect of the present invention, a pharmaceutical composition includes about 10 mg pazopanib/mL of the composition, about 2.0 to about 13.0 % w/w of a modified cyclodextrin, a pH adjusting agent as needed to provide a pH of 3.5 to 5.7, a tonicity adjusting agent as needed to provide an osmolality of 200 to 400 mOsm, and water, where the composition is a super-saturated aqueous solution of pazopanib. The composition is stable for at least 2 months.

In yet another aspect of the present invention, a pharmaceutical composition includes about 10 mg pazopanib/mL of the composition, about 2.0 to about 13.0 % w/w of a modified cyclodextrin, a pH adjusting agent as needed to provide a pH of 3.5 to 5.7, a tonicity adjusting agent as needed to provide an osmolality of 200 to 400 mOsm, and water.

In another aspect of the present invention, a method of preparation of a super-saturated composition includes forming an aqueous solution of an acid addition salt of pazopanib and a modified cyclodextrin, and adjusting the pH of said solution to between about 4 to about 5 to obtain a super-saturated solution of pazopanib that is stable for at least 2 months.

The pharmaceutical compositions according to the various aspects of the invention described herein are preferably suitable for topical administration to the eye of a human. In some embodiments, the pharmaceutical compositions according to the various aspects of the invention described herein are suitable for topical administration as eye drops to the eye of a human. One of skill in the art can use any one of various available methods to determine if the composition is suitable for topical administration to the eye of a human and is suitable for topical administration to the eye of a human as eye drops.

As used herein, the chemical name "pazopanib" refers to the compound 5-[[4-[(2,3-dimethyl-2H-indazol-6-yl)methylamino]-2-pyrimidinyl]amino]-2-methylbenzenesulfonamide, which compound is represented by Structure I:

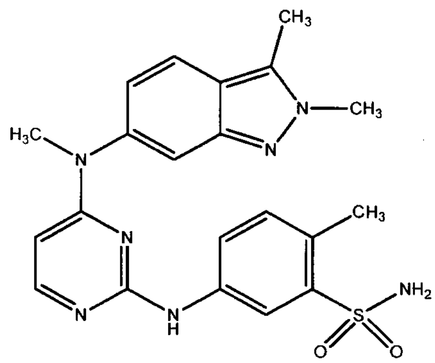

(I).

In some embodiments according to the various aspects of the present invention described herein, the acid addition salt of the compound of formula (I) is a hydrochloride salt. In a particular embodiment, the acid addition salt of the compound of formula (I) is a monohydrochloride salt as illustrated by formula (I').

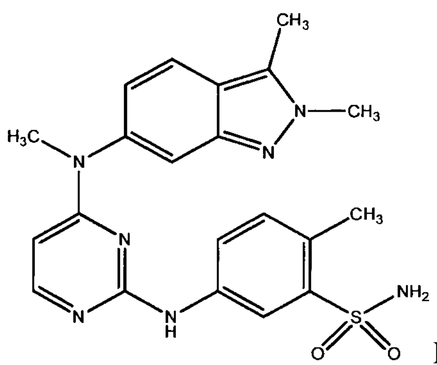

HCl

(I')

The monohydrochloride salt of the compound of formula (I) has the chemical name 5-[[4-[(2,3-dimethyl-2H-indazol-6-yl)methylamino]-2-pyrimidinyl]amino]-2-methylbenzenesulfonamide monohydrochloride.

In other embodiments, the acid addition salt of the compound of formula (I) is a monohydrochloride monohydrate solvate of the compound of formula (I). The monohydrochloride monohydrate solvate of the compound of formula (I) has the chemical name 5-({4-[(2,3-dimethyl-2H-indazol-6-yl)methylamino]-2-pyrimidinyl}amino)-2-methylbenzenesulfonamide monohydrochloride monohydrate, as illustrated in formula (I").

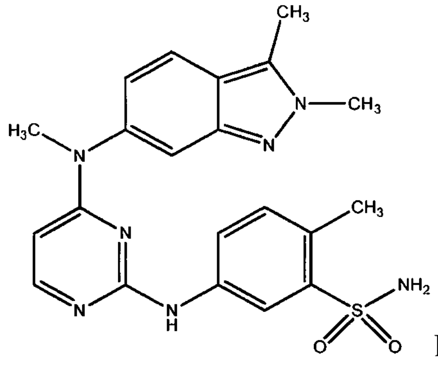

$HCl \cdot H_2O$

(I")

The free base, salts and solvates of the compound of formula (I) may be prepared, for example, according to the procedures of International Patent Application No. PCT/US01/49367 filed December 19, 2001, and published as WO 02/059110 on August 1, 2002, and International Patent Application No. PCT/US03/19211 filed June 17, 2003, and published as WO 03/106416 on December 24, 2003, or according the methods provided herein.

As used herein, the term "acid addition salts" are salts derived from one or more nitrogens on a substituent in the compound of formula (I). Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate.

Pazopanib is a poorly water-soluble drug, having an approximate solubility at 25°C in phosphate buffer as follows: 0.000006 mg/mL ($1.37 \times 10^{-8}$ mol/L) at pH 5.0, 0.000025 mg/mL ($5.71 \times 10^{-8}$ mol/L) at pH 4.5; 0.000534 mg/mL ($1.22 \times 10^{-6}$ mol/L) at pH 4.25; 0.001043 mg/mL ($2.38 \times 10^{-6}$ at pH 4.0; and 0.02 mg/mL ($4.57 \times 10^{-5}$ mol/mL) at pH 3.5.

The cyclodextrin utility number, $U_{CD}$, is a dimensionless number used to assess the feasibility of using cyclodextrin in dosage forms. $U_{CD}$ is a lumped parameter consisting of the dose of the drug, the workable amount of CD, the binding constant, and the drug solubility in the absence of CDs. $U_{CD}$ can be used to predict the solubility of ionizable drugs showing a synergistic increase in solubility due to ionization and complexation. See, Rao, V.M., Stella, V.J., J Pharm Sci, 92, 5 927, May 2003. $U_{CD}$ is calculated using the following equation:

$$U_{CD} = (KS_o/1+KS_o)(m_{CD}/m_D)(\ MW_D/MW_{CD})$$

where,

- $m_D$ is dose of drug;
- $m_{CD}$ is dose of cyclodextrin;
- $MW_D$ is molecular weight drug;
- $MW_{CD}$ is molecular weight cyclodextrin; and
- $K_o$ is binding constant.

In some embodiments, the $U_{CD}$ value is in the range from a lower limit of about 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.105, 0.11, 0.115, 0.12, 0.125, 0.13, 0.135, 0.14, 0.145, 0.15,

0.155, 0.16, 0.165, 0.17, 0.175, 0.18, 0.185, 0.19, 0.195, 0.2, 0.205, 0.21, 0.215, 0.22, 0.225, 0.23, 0.235, 0.24, 0.245, 0.25, 0.255, 0.26, 0.265, 0.27, 0.275, 0.28, 0.285, 0.29, 0.295, 0.3, 0.305. 0.31, 0.315, 0.32, 0.325, 0.33, 0.335, 0.34, 0.345, 0.35, 0.355, 0.36, 0.365, 0.37, 0.375, 0.38, 0.385, 0.39, 0.395, or 0.4 to an upper limit of about 0.2, 0.205, 0.21, 0.215, 0.22, 0.225, 0.23, 0.235, 0.24, 0.245, 0.25, 0.255, 0.26, 0.265, 0.27, 0.275, 0.28, 0.285, 0.29, 0.295, 0.3, 0.305. 0.31, 0.315, 0.32, 0.325, 0.33, 0.335, 0.34, 0.345, 0.35, 0.355, 0.36, 0.365, 0.37, 0.375, 0.38, 0.385, 0.39, 0.395, 0.4, 0.405, 0.41, 0.415, 0.42, 0.425, 0.43, 0.435, 0.44, 0.445, 0.45, 0.455, 0.46, 0.465, 0.47, 0.475, 0.48, 0.485, 0.49, 0.495, 0.5, 0.505. 0.51, 0.515, 0.52, 0.525, 0.53, 0.535, 0.54, 0.545, 0.55, 0.555, 0.56, 0.565, 0.57, 0.575, 0.58, 0.585, 0.59, 0.595, or 0.6 at a temperature of 25°C.

The modified cyclodextrin is preferably selected such that when in the ophthalmic pharmaceutical composition, the modified cyclodextrin is suitable for administration to the eye of a human. Whether a modified cyclodextrin is suitable for administration to the eye of a human can be determined using any one of various available methods and procedures known to those of skill in the art for determining whether compositions and components are suitable for administration to the eye of a human being.

In some embodiments according to the various aspects of the present invention described herein, the modified cyclodextrin is selected from the group consisting of hydroxypropyl-β-cyclodextrin, methyl-β-cyclodextrin, β-cyclodextrin sulfobutylether and combinations thereof. In other embodiments, the modified cyclodextrin is β-cyclodextrin sulfobutylether. A β-cyclodextrin sulfobutylether is sold under the tradename Captisol®.

In some embodiments according to the various aspects of the present invention described herein, the amount of the modified cyclodextrin is in the range of a lower limit of about 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0 % w/w to an upper limit of about 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0, 12.1, 12.2, 12.3, 12.4, 12.5, 12.6, 12.7, 12.8, 12.9 or 13.0 % w/w.

The United States Pharmacopeia (USP) <1151> entitled "Pharmaceutical Dosage Forms Ophthalmic Preparations" provides the following guidance regarding the isotonicity of ophthalmic solutions.

Regarding isotonicity values, USP < 1151 > states that lacrimal fluid is isotonic with blood, having an isotonicity value corresponding to that of a 0.9% sodium chloride solution. Ideally, an ophthalmic solution should have this isotonicity value; but the eye can tolerate isotonicity values as low as that of a 0.6% sodium chloride solution and as high as that of a 2.0% sodium chloride solution without marked discomfort. These values roughly correspond to osmolality values of 200 mOsm for a 0.6% w/v NaCl solution in water, 290 mOsm for a 0.9% w/v NaCl solution in water, and 631 mOsm for a 2.0% w/v NaCl solution in water. However, a survey of FDA approved ophthalmic products including Timoptic-Xe® (timilol maleate ophthalmic gel forming solution), Voltaren® (diclofenac sodium ophthalmic solution), Aphagen® (brimonidine tartrate ophthalmic solution), Ocufen® (flurbiprofen sodium ophthalmic solution, USP), Travatan Z® (travaprost ophthalmic solution), Vitoptic® (trifluridine ophthalmic solution), Alamast® (pemirolast potassium ophthalmic solution), Vigamox® (moxifloxacin hydrochloride ophthalmic solution), and Poly-Pred® (prednisolone acetate, neomycin sulfate, polymyxin B sulfate ophthalmic solution, USP) reveals that these approved products have osmolalities in the range of a low of 240 mOsm to a high of 350 mOsm, with most being centered around 290 mOsm. Thus, it would be desirable to have an ophthalmic formulation having an osmolality in the range of about 200 to about 400 mOsm.

In some embodiments according to the various aspects of the present invention described herein, the osmolality of the composition is in the range of a lower limit of about 200, 210, 220, 230, 240, 250, 260, 270, 280, 290, 300, 310, 320, or 330 mOsm to an upper limit of about 250, 260, 270, 280, 290, 300, 310, 320, 330, 340, 350, 360, 370, 380, 390 or 400 mOsm. Modified cyclodextrins contribute to the tonicity of the pharmaceutical composition. For example, applicants have found that Captisol® (β-cyclodextrin sulfobutylether) contributes to the osmolality of the solution in the following manner:

**Table 1**

| Captisol % (w/v) | Osmolality (mOsm) |
|---|---|
| 2 | 20 |
| 3 | 50 |
| 4 | 80 |
| 5 | 110 |
| 6 | 140 |
| 7 | 170 |

(continued)

| Captisol % (w/v) | Osmolality (mOsm) |
|---|---|
| 8 | 201 |
| 9 | 231 |
| 10 | 261 |
| 11 | 291 |
| 12 | 321 |
| 13 | 351 |

The fact that modified cyclodextrins contribute to the osmolality and that osmolality needs to be 400 mOsm or below imposes an upper limit on the amount of modified cyclodextrin that can be used to solubilize the pazopanib in the pharmaceutical composition.

Tonicity is the effective osmolality and is equal to the sum of the concentrations of the solutes which have the capacity to exert an osmotic force across the membrane. The tonicity adjusting agent used in embodiments of the present invention may be any of various such agents known to those of skill in the art to be suitable for inclusion in a composition for ocular administration to the human eye. For example, the United States Pharmacopeia 29-NF-24 lists five excipients classified as "tonicity" agents including dextrose, glycerin, mannitol, potassium chloride and sodium chloride. Those of skill in the art will understand, of course, that other excipients can be used in the formulation as tonicity adjusting agents. For example, buffering agents such as phosphate buffers (e.g., sodium phosphate or potassium phosphate buffers) not only buffer the pH of the solution, but also act as tonicity adjusting agents. In some embodiments, the tonicity adjusting agent is selected from the group consisting of dextrose, glycerin, mannitol, potassium chloride, sodium chloride, and phosphate buffers. In general, the amount of the tonicity agent will be enough to provide an osmolality of the pharmaceutical composition that is from a lower limit of 200 to an upper limit of 400 mOsm. In some embodiments, the tonicity adjusting agent is sodium chloride. In some embodiments, the amount of sodium chloride is in the range of a lower limit of 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 mM to an upper limit of about 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, or 150 mM. Amounts of other tonicity adjusting agents can be the amount of the particular tonicity adjusting agent required to provide similar contributions to osmolality as that provided by the described amounts of sodium chloride. In some embodiments, combinations of tonicity adjusting agents are used.

In some embodiments of the present invention, the pharmaceutical composition also includes a buffering agent. The buffering agent can be any of various buffering agents known to those of skill in the art to be useful for ophthalmic formulations. For example, the buffering agent can be a phosphate buffer, such as sodium phosphate or potassium phosphate. In some embodiments, the amount of the buffering agent is in the range of a lower limit of 10, 11, 12, 13, 14 ,15 16, 17, 18, 19, 20, 21, 22, 23, 24, 25, 26, 27, 28, 29, 30, 31, 32, 33, 34, 35, 36, 37, 38, 39 or 40 mM to an upper limit of about 30, 31, 32, 33, 34, 35, 36, 37, 38, 39, 40, 41, 42, 43, 44, 45, 46, 47, 48, 49 or 50 mM. In some embodiments, combinations of buffering agents are used.

The pH adjusting agent may be various such agents known to those of skill in the art to be suitable for inclusion in a composition for ocular administration to the human eye. In some embodiments according to the various aspects of the present invention, the pH adjusting agent is selected from the group consisting of sodium hydroxide, hydrochloric acid and combinations thereof.

The United States Pharmacopeia (USP) <1151> entitled "Pharmaceutical Dosage Forms Ophthalmic Preparations" provides the following guidance regarding the pH of ophthalmic solutions. Normal tears have a pH of about 7. In some cases pH of ophthalmic solutions may vary between 3.5 and 8.5. Thus, it would be desirable to have an ophthalmic formulation with a pH in the range of 3.5 to 8.5.

In some embodiments according to the various aspects of the present invention described herein, the pH of the composition is in the range of a lower limit of about 3.5, 3.55, 3.6, 3.65, 3.7, 3.75, 3.8, 3.85, 3.9, 3.95 , 4.0, 4.05, 4.1, 4.15, 4.2, 4.25, 4.3, 4.35, 4.4, 4.45, 4.5, 4.55, 4.6, 4.65, 4.7, 4.75, 4.8, 4.85, 4.9, 4.95, or 5.0 to an upper limit of about 5.0, 5.05, 5.1, 5.15, 5.2, 5.25, 5.3, 5.35, 5.4, 5.45, 5.5, 5.55, 5.6, 5.65, or 5.7. Although USP <1151> indicates that pH up to 8.5 can be used for ophthalmic formulations, the solubility of pazopanib varies with pH, with solubility decreasing as pH increases. Thus, for pH levels above 5.7, the solubility of pazopanib is so low that the amount of

modified cyclodextrin cannot be increased to a sufficient level to solubilize the pazopanib without increasing the tonicity of the formulation to a level that is above the desired upper level of 400 mOsm.

In some embodiments according to the various aspects of the present invention described herein, the composition is stable for at least 2, 3, 4, 5, 6, 7, 8, 9, 10, 11, 12 or more months.

The following examples are intended for illustration only and are not intended to limit the scope of the invention in any way.

## EXAMPLES

### Example 1: Composition

A composition of pazopanib monohydrochloride solution is given below in Table 2. The pH can be adjusted to provide the desired pH, such as a pH in the range of about 3.5 to about 5.7.

**Table 2**

| Component | 10 mg/mL | Function |
|---|---|---|
| Pazopanib hydrochloride (mg/mL) | 10.834 | Active ingredient pazopanib in the form of the monohydrochloride salt |
| β-cyclodextrin Sulfobutylether (mg/mL) | 90.000 | Solubility enhancer |
| Monobasic Sodium Phosphate (mg/mL) | 3.450 | Buffering agent |
| Water for Injection | q.s. | Solvent |
| Sodium Hydroxide | q.s. | pH adjustment |
| Hydrochloric Acid | q.s. | pH adjustment |

### Example 2: Method of Preparing 10 mg/mL Composition Pazopanib Monohydrochloride Solution

A formulation as described in Example 1 above is prepared by a method as described in this Example 2. Manufacture of the bulk solution is conducted in a Grade C environment. β-cyclodextrin sulfobutylether (e.g., Captisol® from Cydex Pharmaceuticals, Inc., Lenexa, KS) is added to an appropriate vessel containing water for injection (WFI) and gently mixed until visibly dissolved. The following ingredients are then individually added to the vessel in order, gently mixed and allowed to dissolve before proceeding to the next addition: active ingredient (pazopanib in the form of the monohydrochloride salt), and monobasic sodium phosphate, monohydrate. The solution is brought to volume with WFI and gently mixed. The pH of the solution is adjusted to the desired pH, such as a pH of about 3.5 to about 5.7, if necessary, using 1 N hydrochloric acid or 1 N sodium hydroxide solution. The resulting solution is filtered using two sterile 0.22 μm sterilizing filters (in series), followed by a third 0.22 μm filter within the blow-fill-seal equipment, prior to filling.

**Flow Diagram of a Manufacturing Process of Pazopanib hydrochloride Solution, 10 mg/mL**

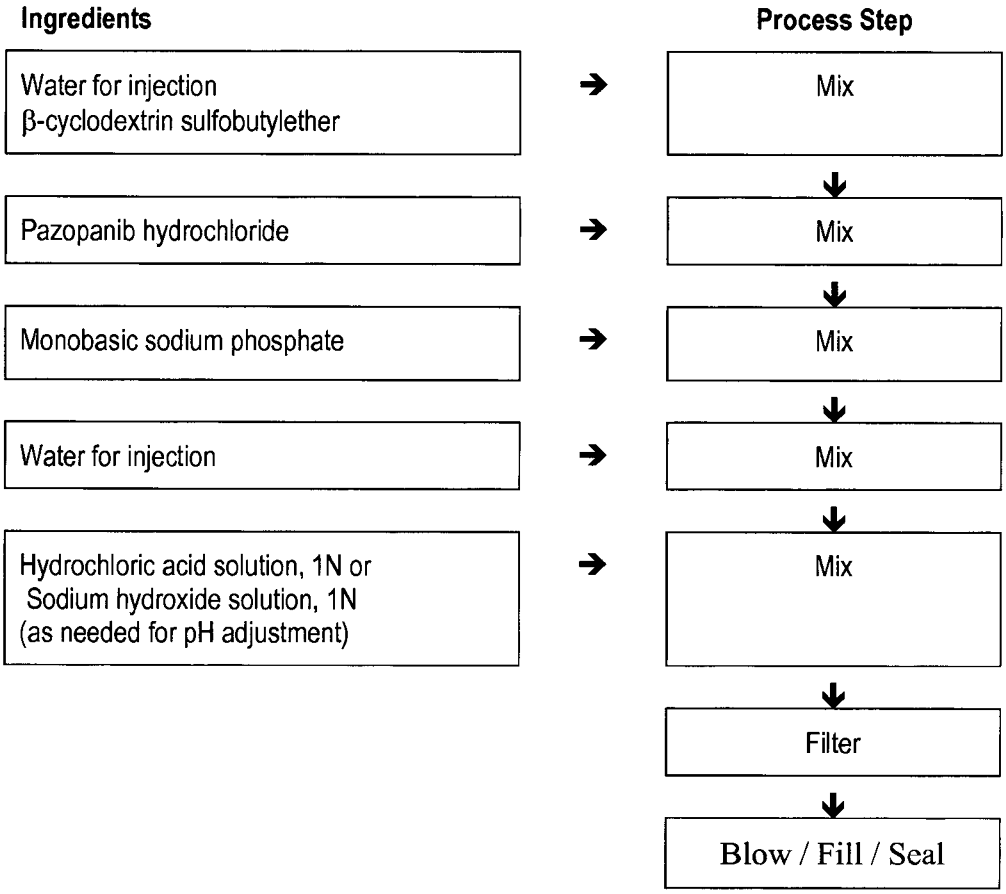


**Example 3: Determining Stability of a 10 mg Pazopanib/mL Composition**

For the purposes of this example 3, the physical stability of a pazopanib monohydrochloride composition of 10 mg pazopanib/mL (the "label claim") such as the composition described above in Example 1 packaged in a blow-fill sealed single-use container that is overwrapped using blank flow wrap aluminum foil is determined by measuring the clarity, color, pH, pazopanib content as percent of the label claim, particle count ≥ 10 μm, and particle count ≥ 25 μm of the composition.

**Methods**

**Clarity and Color**

Equipment

i. Vials: Identical vials of colorless, transparent, neutral glass
ii. Pipette: Glass, Class A or Digital
iii. Test Tubes: 13 mm x 100 mm, borosilicate, (Baxter T-1290-4)
iv. Fluorescent Light Source: Macbeth SpectraLight II Booth
v. White/Black Board

Reagents

i. Concentrated Hydrochloric Acid (HCl): Analytical Grade (37%)
ii. Cobaltous Chloride (CoCl2 .6H20): Reagent Grade
iii. Cupric Sulphate (CuSO4.5H20): Reagent Grade
iv. Ferric Chloride (FeCl3.6H20): Reagent Grade
v. Water, deionised: Barnstead NanoPure

Solutions

*Stock Solutions*

1%w/v Hydrochloric Acid Solution: Dilute 135 mL of concentrated HCl to 5 liters with water.

Red Solution (Cobaltous Chloride, CS)

Blue Solution (Cupric Sulfate, CS)

Yellow Solution (Ferric Chloride, CS)

Proceed according to the current USP under Reagents, Solution, Colorimetric Solutions (CS). Store the solutions in suitably resistant, tight containers and store at 5°C in the dark. CS solutions are restandardized every 12 months. These solutions are used in the preparation of the colorimetric standards for color comparison as per USP <631> Color and Achromicity.

Colorimetric Standard Solutions

Using the three Stock CS solutions from above, prepare the five standard solutions listed below. Store the standard solutions in tightly sealed glass containers. These solutions are stable for twelve months when stored at 5°C in the dark.

**Standard Solutions**

| | Yellow Solution (mL) | Red Solution (mL) | Blue Solution (mL) | 1%w/v HC1 (mL) |
|---|---|---|---|---|
| B (brown) | 60.0 | 60.0 | 48.0 | 32.0 |
| BY (brownishyellow) | 48.0 | 20.0 | 8.0 | 124.0 |
| Y (yellow) | 48.0 | 12.0 | 0.0 | 140.0 |
| GY (greenishyellow) | 192.0 | 4.0 | 4.0 | 0.0 |
| R (red) | 20.0 | 40.0 | 0.0 | 140.0 |

Reference Solutions

As defined in the Tables below, transfer an aliquot from one of the five standard solutions listed in Section 4.2 (above) to individual 50-mL volumetric flasks. Dilute to 50 mL with 1% HCl. Store the reference solutions in tightly sealed glass containers. The reference solutions are stable for twelve months at room temperature.

| Reference Solution | Standard Solution B (mL) |
|---|---|
| B1 | 37.5 |
| B2 | 25.0 |
| B3 | 18.75 |

(continued)

| Reference Solution | Standard Solution B (mL) |
|---|---|
| B4 | 12.5 |
| B5 | 6.25 |
| B6 | 2.5 |
| B7 | 1.25 |
| B8 | 0.75 |
| B9 | 0.5 |

| Reference solution | Standard Solution BY (mL) |
|---|---|
| BY1 | 50.0 |
| BY2 | 37.5 |
| BY3 | 25.0 |
| BY4 | 12.5 |
| BY5 | 6.25 |
| BY6 | 2.5 |
| BY7 | 1.25 |

| Reference solution | Standard Solution Y (mL) |
|---|---|
| Y1 | 50.0 |
| Y2 | 37.5 |
| Y3 | 25.0 |
| Y4 | 12.5 |
| Y5 | 6.25 |
| Y6 | 2.5 |
| Y7 | 1.25 |

| Reference solution | Standard Solution GY (mL) |
|---|---|
| GY1 | 12.5 |
| GY2 | 7.5 |
| GY3 | 4.25 |
| GY4 | 2.5 |
| GY5 | 1.5 |
| GY6 | 0.75 |
| GY7 | 0.375 |

| Reference Solution | Standard Solution R (mL) |
|---|---|
| R1 | 50.0 |
| R2 | 37.5 |
| R3 | 25.0 |
| R4 | 18.75 |
| R5 | 12.5 |
| R6 | 6.25 |
| R7 | 2.5 |

Methods

All dosage units are visually inspected and the uniformity across the entire sample is noted. If the entire sample appears uniform, one sample is selected for detailed examination. If the entire sample is not uniform, two samples, representing the extremes of each observed range of variation, are selected for detailed examination.

**Clarity**

The degree of clarity of the solution is determined when a sample of the solution is compared to an equal volume of water in a similar container using both a white and black background.

**Color**

Comparison of colors as directed in the Pharmacopoeial tests preferably is made in matched color-comparison tubes or in a suitable colorimeter under conditions that ensure that the colorimetric reference solution and that of the specimen under test are treated alike in all respects. The comparison of colors is best made in layers of equal depth, and viewed transversely against a white background under fluorescent light. It is particularly important that the solutions be compared at the same temperature, preferably 25°C. The color of a solution is determined when a sample of the solution is compared to an equal volume of water in a similar container. If there is no difference, the solution is reported as colorless. If the solution differs from water, compare it to the reference solutions B9, BY7, Y7, GY7, and R7; report as colorless if the sample is lighter than these reference solutions. Otherwise, select the reference solution, which is comparable to the sample solution and compare to the other reference solutions in the same group (e.g., Y1, Y2, Y3, Y4, Y5, Y6, and Y7) to bracket the solution under test. Record the identity of the reference solution that most closely matches the sample. If the sample's color is between two reference solutions, report the result as between the values of the two reference solutions.

"Colorless" is reported for solutions that exhibit no color (same as water) or exhibit color lighter than reference solutions B9, BY7, Y7, GY7, or R7.

**pH**

pH is measured by a suitable method as will be understood by those skilled in the art.

**Pazopanib Content (% label claim)**

Equipment

**Balance:** Mettler MT5 or MX5
**Chromatography:** An HPLC system capable of performing gradient elution equipped with a variable-wavelength UV detector
**Column:** Phenomenex Develosil RPAqueous-3, C-30, 3 um, 150 mm x 4.6 mm
**Data Acquisition System:** Atlas or Empower
**Filter:** Millex-GV PVDF, 0.22 μm, 33 mm diameter, sterile syringe filter; or Acrodisc Nylon, 0.2 μm, 25 mm diameter syringe filter

**Syringe:** Becton and Dickinson 10 mL plastic

Reagents

**Pazopanib:** Analytical Reference Standard
**Acetonitrile (ACN):** HPLC grade
**Trifluoroacetic acid (TFA):** HPLC grade
**Water:** De-ionized water, Milli-Q or HPLC grade

Preparation of Solutions

**Preparation of Dissolving Solvent**

Prepare a mixture of ACN:Water:TFA in the ratio 50:50:0.1 by volume. For example, mix 1 mL of TFA and 500 mL of water well. Add 500 mL of acetonitrile and mix well.

**Preparation of Mobile Phase A**

Prepare a mixture of Water:ACN:TFA in the ratio 90.5:9.5:0.1 by volume. For example, mix well 1 mL TFA and 905 mL of Water. Add 95 mL ACN and mix well. Degas before use.

**Preparation of Mobile Phase B**

Prepare a mixture of Water:ACN:TFA in the ratio 40.5:59.5:0.1 by volume. For example, mix well 1 mL TFA and 405 mL of Water. Add 595 mL ACN and mix well. Degas before use.

**Preparation of Standard Solution (in duplicate)**

Prepare 0.1 mg/mL (as free base) of GW786034B reference standard solution in dissolving solvent by transferring approximately 10.8 $\pm$ 1.1mg of GW786034B analytical reference standard (adjust weight for purity as required), accurately weighed into a 100 mL volumetric flask. Do not leave weigh boat in flask. Dilute to approximately 2/3 full with dissolving solvent and sonicate at least 5 minutes or until dissolved. Allow to equilibrate to room temperature, then dilute to volume with dissolving solvent. The standard solution is stable for at least 40 days at either 5°C or room temperature without protection from laboratory light.

**NOTE:** Other preparation schemes can be used which result in a comparable final concentration.

**Preparation of Sensitivity Check Solution**

Dilute 5 mL of standard solution to 100 mL with dissolving solvent. Further dilute 1 mL aliquot of this solution to 100 mL in a volumetric flask to yield a 0.05% (v/v) sensitivity check standard solution. This solution is stable for at least 8 days at room temperature without protection from laboratory light.

**NOTE:** Other preparation schemes can be used which result in a comparable final concentration.

**Preparation of Working Active and Placebo Sample Solutions**

Prepare sample solutions in duplicate using separate stock solutions.

**Preparation of Eye Drop Solution**

Sample Preparation Scheme

| Dosage Strength | Aliquot Taken for Dilution | Dilution Volume | Target Final Concentration |
|---|---|---|---|
| 10 mg/mL | 2 mL | 200 mL | 0.1 mg/mL |

The working sample solution of the 2 mg/mL preparation is stable for at least 2 days at room temperature without protection from laboratory light. The working sample solution of the 5 mg/mL, 8 mg/mL, 10 mg/mL, and 12 mg/mL preparations is stable for at least 7 days at room temperature without protection from laboratory light.
**NOTE:** Other equivalent sample solution preparation procedures, which yield the same final concentration or a concentration within the linear range of the method, may be used.

Experimental Procedure

**Instrument Parameters**

| | | | |
|---|---|---|---|
| Column | Phenomenex Develosil RP-Aqueous, 3μm, 150 x 4.6mm | | |
| Column temperature | 35 C | | |
| Flow rate | 1.0 mL/min | | |
| Gradient profile | Time (min) | %A | %B |
| | 0 | 100 | 0 |
| | 1 | 100 | 0 |
| | 31 | 10 | 90 |
| | 33 | 100 | 0 |
| | 40 | 100 | 0 |
| Detector wavelength | 268 nm | | |
| Injection volume | 10 μL | | |
| Run time | 40 minutes | | |

**Assay Procedure**

Inject the blank solution(s), standard, and sample solution(s). Following the analysis, the column should be flushed with a 50:50 acetonitrile:water mixture for at least 30 minutes prior to storage. The column should be stored in 50:50 acetonitrile:water. If possible, a needle wash vial should be used to minimize the carryover of pazopanib between injections. Any peaks found in the HPLC blank chromatograms should not prevent the integration of reportable peaks of interest and should be excluded from the sample chromatograms. Carryover of pazopanib in the blank chromatograms should be less than or equal to 0.1%. If high carryover persists rinse all lines of the HPLC system as well as the HPLC injector with Isopropyl Alcohol.

**Identity Testing**

The identity of a sample is confirmed if the retention time of the pazopanib peak in the Sample Solution is within ±3% of the retention time of the pazopanib peak in the Reference Standard chromatogram. The identity of the placebo solution is confirmed either by the absence of a peak at the same retention time as seen for the principal peak in the chromatograms of the pazopanib reference material, or if the calculated strength in the placebo sample is less than or equal to 0.1% of a 2 mg/mL active dose.

**Calculation of Pazopanib Content**

Response Factor, K

$$K = (PxSxC_S)/A_S$$

where:

As = The peak area response for a single injection of Standard Solution.
P = Purity of GW786034B reference standard (in decimal terms).
S = The factor for converting the hydrochloride salt of pazopanib into free base equivalents (i.e., 0.923).
Cs = Concentration of pazopanib in Standard Solution (mg/mL).

% Label Claim

$$Au \times Kav \times Du \times (1/L) \times 100 = \% \text{ Label Claim}$$

where:

Au = the peak area response of the Sample Solution.
Kav = the average Response Factor for all injections of the Standard Solution.
Du = the dilution factor for the Sample Solution
L = label claim

**Particle Count**

Method A

Particle Count is determined according to United States Pharmacopeia (USP) <789> "Particulate Matter in Ophthalmic Solutions".

Method B

Equipment

**Particle Counter:** HIAC-Royco 9703
**Data Acquisition System:** PharmSpec

Reagents

**Water:** particle-free deionised water
Preparation of Solutions

Refer to USP<789>, Particulate Matter in Ophthalmic Solutions for all the sample preparation procedures.

Procedure of Gas Bubbles Elimination for Eye Drop Solution

Prior to the particulate testing, each eye drop sample solution must be degassed by means of a vacuum desiccator following the procedure below:

1. Loosely cover the bulk sample container with a lid and place into a vacuum desiccator.
2. Close the desiccator lid and ensure that the release valve is open.
3. Attach a calibrated gauge to the desiccator and attach the desiccator to a vacuum tap.
4. Turn the vacuum tap on until a gauge pressure of -20 to -25 inches of Hg is achieved, then turn off the vacuum tap. May need to turn the vacuum tap on and off periodically to keep the vacuum pressure in the range of -20 to -25 inches of Hg during the vacuum application.

5. After 5 minutes, carefully release the vacuum.
6. Once normal pressure has been re-established, open the desiccator lid.
7. Perform the particulate testing immediately.

The results of the particle count should be reported as number of particles per mL. Particle count /mL = (average of the number of particles (≥ 10 μm or ≥ 25 μm))/volume where: volume = 5 mL

Method C

Method C is similar to Method B above, except particular care may be taken (e.g. gloves can be rinsed with particle free water prior to sample prep; sample containers can be rinsed with particle free water only; submerge the sampling needle in the particle free water when it is not in use) in order to avoid any particle contamination during sample preparation and potential introduction of air into the sampling needle.

## Results

The physical stability of a pazopanib monohydrochloride composition of 10 mg pazopanib/mL (the "label claim") such as the composition described above in Example 1 at pH 4.0 is shown in Table 4.

**Table 4**

| Storage Conditions | Time (months) | Sample No. | Clarity | Color | pH | Pazopanib Content (% label claim) | Particle Count ≥ 10 μm (Method) | Particle Count ≥ 25 μm (Method) |
|---|---|---|---|---|---|---|---|---|
| Initial | 0 | DG0001-1 | Clear | Colorless | 4.15 | 104.2 | 0.0 (A) | 0.0 (A) |
| | 0 | DG0001-2 | DNM | DNM | DNM | 104.4 | DNM | DNM |
| 5°C/ambient relative humidity (RH) (packaged in (packaged in blow-filled seal container) | 1 | DG0001-5/amb-1 | Clear | Colorless | 4.09 | 103.9 | 0.2 (A) | 0.0 (A) |
| | 1 | DG0001-5/amb-2 | DNM | DNM | DNM | 104.2 | DNM | DNM |
| | 3 | DG0001-5/amb-1 | Clear | Colorless | 4.04 | 104.1 | 2.7 (B) | 0.0 (B) |
| | 3 | DG0001-5/amb-2 | DNM | DNM | DNM | 104.0 | DNM | DNM |
| | 6 | DG0001-5/amb-1 | Clear | Colorless | 4.04 | 103.5 | 0.2 (C) | 0.1 (C) |
| | 6 | DG0001-5/amb-2 | DNM | DNM | DNM | 103.5 | DNM | DNM |
| | 9 | DG0001-5/amb-1 | Clear | Colorless | 4.08 | 104.1 | 0.3 (C) | 0.0 (C) |
| | 9 | DG0001-5/amb-2 | DNM | DNM | DNM | 104.7 | DNM | DNM |
| | 12 | DG0001-5/amb-1 | Clear | Colorless | 4.05 | 103.5 | 1.7 (C) | 0.0 (C) |
| | 12 | DG0001-5/amb-2 | DNM | DNM | DNM | 102.8 | DNM | DNM |
| | 14 | DG0001-5/amb-1 | DNM | DNM | DNM | DNM | DNM | DNM |
| | 14 | DG0001-5/amb-2 | DNM | DNM | DNM | DNM | DNM | DNM |

(continued)

| Storage Conditions | Time (months) | Sample No. | Clarity | Color | pH | Pazopanib Content (% label claim) | Particle Count ≥ 10 μm (Method) | Particle Count ≥ 25 μm (Method) |
|---|---|---|---|---|---|---|---|---|
| 25°C/60% RH (packaged in (packaged in blow-filled seal container) | 1 | DG0001-25/60-1 | Clear | Colorless | 4.11 | 103.8 | 0.5 (A) | 0.1 (A) |
| | 1 | DG0001-25/60-2 | DNM | DNM | DNM | 103.6 | DNM | DNM |
| | 3 | DG0001-25/60-1 | Clear | Colorless | 4.04 | 104.7 | 7.8 (B) | 0.3 (B) |
| | 3 | DG0001-25/60-2 | DNM | DNM | DNM | 104.1 | DNM | DNM |
| | 6 | DG0001-25/60-1 | Clear | Colorless | 4.06 | 104.8 | 0.2 (C) | 0.0 (C) |
| | 6 | DG0001-25/60-2 | DNM | DNM | DNM | 103.0 | DNM | DNM |
| | 9 | DG0001-25/60-1 | Clear | Colorless | 4.07 | 106.3 | 0.1 (C) | 0.0 (C) |
| | 9 | DG0001-25/60-2 | DNM | DNM | DNM | 104.8 | DNM | DNM |
| | 12 | DG0001-25/60-1 | Clear | Colorless | 4.05 | 104.1 | 0.1 (C) | 0.0 (C) |
| | 12 | DG0001-25/60-2 | DNM | DNM | DNM | 104.1 | DNM | DNM |
| | 14 | DG0001-25/60-1 | DNM | DNM | DNM | DNM | DNM | DNM |
| | 14 | DG0001-25/60-2 | DNM | DNM | DNM | DNM | DNM | DNM |

(continued)

| Storage Conditions | Time (months) | Sample No. | Clarity | Color | pH | Pazopanib Content (% label claim) | Particle Count ≥ 10 μm (Method) | Particle Count ≥ 25 μm (Method) |
|---|---|---|---|---|---|---|---|---|
| 40°C/25% RH (packaged in blow-filled seal container) | 1 | DG0001-40/25-1 | Clear | Colorless | 4.11 | 105.4 | 0.1 (A) | 0.0 (A) |
| | 1 | DG0001-40/25-2 | DNM | DNM | DNM | 103.8 | DNM | DNM |
| | 3 | DG0001-40/25-1 | Clear | Colorless | 4.03 | 105.4 | 7.9 (B) | 0.1 (B) |
| | 3 | DG0001-40/25-2 | DNM | DNM | DNM | 105.0 | DNM | DNM |
| | 6 | DG0001-40/25-1 | Clear | Colorless | 4.07 | 107.0 | 0.1 (C) | 0.0 (C) |
| | 6 | DG0001-40/25-2 | DNM | DNM | DNM | 106.4 | DNM | DNM |
| | 9 | DG0001-40/25-1 | Clear | Colorless | 4.07 | 106.8 | 0.1 (C) | 0.0 (C) |
| | 9 | DG0001-40/25-2 | DNM | DNM | DNM | 105.6 | DNM | DNM |
| | 12 | DG0001-40/25-1 | DNM | DNM | DNM | DNM | DNM | DNM |
| | 12 | DG0001-40/25-2 | DNM | DNM | DNM | DNM | DNM | DNM |
| | 14 | DG0001-40/25-1 | Clear | Colorless | 4.08 | 105.9 | 0.5 (C) | 0.0 (C) |
| | 14 | DG0001-40/25-2 | DNM | DNM | DNM | 106.5 | DNM | DNM |

The physical stability of a pazopanib monohydrochloride composition of 10 mg pazopanib/mL (the "label claim") such as the composition described above in Example 1 at pH 4.25 is shown in Table 5.

**Table 5**

| Storage Conditions | Time (months) | Sample No. | Clarity | Color | pH | Pazopanib Content (% label claim) | Particle Count ≥ 10 μm | Particle Count ≥ 25 μm |
|---|---|---|---|---|---|---|---|---|
| **Initial** | 0 | BD0001-1 | Clear | Colorless | 4.32 | 103.9 | 0.2 (A) | 0.1 (A) |
| | 0 | BD0001-2 | DNM | DNM | DNM | 102.6 | DNM | DNM |
| **5°C/ambient relative humidity (RH) (packaged in blow-filled seal container)** | 1 | BD0001 - 5/amb-1 | DNM | DNM | DNM | 102.7 | DNM | DNM |
| | 1 | BD0001-5/amb-2 | Clear | Colorless | 4.29 | 104.2 | 2.8 (B) | 0.0 (B) |
| | 3 | BD0001-5/amb-1 | DNM | DNM | DNM | 102.9 | DNM | DNM |
| | 3 | BD0001-5/amb-2 | Clear | Colorless | 4.31 | 102.9 | 0.7 (B) | 0.1 (B) |
| | 4.5 | BD0001-5/amb-1 | DNM | DNM | DNM | 102.5 | DNM | DNM |
| | 4.5 | BD0001-5/amb-2 | Clear | Colorless | 4.31 | 102.7 | 0.7 (C) | 0.0 (C) |
| | 6 | BD0001-5/amb-1 | Clear | Colorless | 4.28 | 103.9 | 0.7 (C) | 0.0 (C) |
| | 6 | BD0001-5/amb-2 | DNM | DNM | DNM | 105.0 | DNM | DNM |
| | 9 | BD0001-5/amb-1 | DNM | DNM | DNM | 102.9 | DNM | DNM |
| | 9 | BD0001-5/amb-2 | Clear | Colorless | 4.30 | 103.9 | 0.7 (C) | 0.1 (C) |
| | 12 | BD0001-5/amb-1 | Clear | Colorless | 4.28 | 104.5 | 1.5 (C) | 0.0 (C) |
| | 12 | BD0001-5/amb-2 | DNM | DNM | DNM | 104.3 | DNM | DNM |

(continued)

| Storage Conditions | Time (months) | Sample No. | Clarity | Color | pH | Pazopanib Content (% label claim) | Particle Count ≥ 10 μm | Particle Count ≥ 25 μm |
|---|---|---|---|---|---|---|---|---|
| **25°C/60% RH (packaged in blow-filled seal container)** | 1 | BD0001-25/60-1 | DNM | DNM | DNM | 102.9 | DNM | DNM |
| | 1 | BD0001-25/60-2 | Clear | Colorless | 4.29 | 102.7 | 3.3 (A) | 0.1 (A) |
| | 3 | BD0001-25/60-1 | DNM | DNM | DNM | 104.6 | DNM | DNM |
| | 3 | BD0001-25/60-2 | Clear | Colorless | 4.29 | 103.8 | 0.6 (B) | 0.0 (B) |
| | 4.5 | BD0001-25/60-1 | DNM | DNM | DNM | 103.4 | DNM | DNM |
| | 4.5 | BD0001-25/60-2 | Clear | Colorless | 4.29 | 103.5 | 0.5 (C) | 0.0 (C) |
| | 6 | BD0001-25/60-1 | Clear | Colorless | 4.28 | 106.6 | 0.6 (C) | 0.1 (C) |
| | 6 | BD0001-25/60-2 | DNM | DNM | DNM | 104.9 | DNM | DNM |
| | 9 | BD0001-25/60-1 | DNM | DNM | DNM | 105.3 | DNM | DNM |
| | 9 | BD0001-25/60-2 | Clear | Colorless | 4.32 | 105.6 | 0.6 (C) | 0.0 (C) |
| | 12 | BD0001-25/60-1 | Clear | Colorless | 4.27 | 105.6 | 1.0 (C) | 0.0 (C) |
| | 12 | BD0001-25/60-2 | DNM | DNM | DNM | 105.8 | DNM | DNM |

(continued)

| Storage Conditions | Time (months) | Sample No. | Clarity | Color | pH | Pazopanib Content (% label claim) | Particle Count ≥ 10 μm | Particle Count ≥ 25 μm |
|---|---|---|---|---|---|---|---|---|
| **40°C/25% RH (packaged in blow-filled seal container)** | 1 | BD0001-40/25-1 | DNM | DNM | DNM | 103.5 | DNM | DNM |
| | 1 | BD0001-40/25-2 | Clear | Colorless | 4.28 | 104.1 | 2.0 (A) | 0.0 (A) |
| | 3 | BD0001-40/25-1 | DNM | DNM | DNM | 104.5 | DNM | DNM |
| | 3 | BD0001-40/25-2 | Clear | Colorless | 4.28 | 104.1 | 0.0 (B) | 0.0 (B) |
| | 4.5 | BD0001-40/25-1 | DNM | DNM | DNM | 105.1 | DNM | DNM |
| | 4.5 | BD0001-40/25-2 | Clear | Colorless | 4.30 | 105.7 | 0.2 (C) | 0.0 (C) |
| | 6 | BD0001-40/25-1 | Clear | Colorless | 4.27 | 107.8 | 0.1 (C) | 0.0 (C) |
| | 6 | BD0001-40/25-2 | DNM | DNM | DNM | 106.2 | DNM | DNM |
| | 9 | BD0001-40/25-1 | DNM | DNM | DNM | 107.2 | DNM | DNM |
| | 9 | BD0001-40/25-2 | Clear | Colorless | 4.30 | 106.9 | 1.7 (C) | 0.0 (C) |
| | 12 | BD0001-40/25-1 | DNM | DNM | DNM | DNM | DNM | DNM |
| | 12 | BD0001-40/25-2 | DNM | DNM | DNM | DNM | DNM | DNM |

The physical stability of a pazopanib monohydrochloride composition of 10 mg pazopanib/mL (the "label claim") such as the composition described above in Example 1 at pH 4.5 is shown in Table 6.

**Table 6**

| Storage Conditions | Time (months) | Sample No. | Clarity | Color | pH | Pazopanib Content (% label claim) | Particle Count ≥ 10 μm | Particle Count ≥ 25 μm |
|---|---|---|---|---|---|---|---|---|
| **Initial** | 0 | DJ0001-1 | Clear | Colorless | 4.62 | 104.7 | 2.1 (A) | 0.1 (A) |
| | 0 | DJ0001-2 | DNM | DNM | DNM | 103.7 | DNM | DNM |
| **5°C/ambient relative humidity ( RH) (packaged in blow-filled seal container)** | 1 | DJ0001-5/amb-1 | Clear | Colorless | 4.57 | 103.5 | 14.5 (A) | 0.3 (A) |
| | 1 | DJ0001-5/amb-2 | DNM | DNM | DNM | 103.3 | DNM | DNM |
| | 3 | DJ0001-5/amb-1 | Clear | Colorless | 4.49 | 103.9 | 0.8 (B) | 0.2 (B) |
| | 3 | DJ0001-5/amb-2 | DNM | DNM | DNM | 103.1 | DNM | DNM |
| | 6 | DJ0001-5/amb-1 | Clear | Colorless | 4.49 | 104.1 | 0.5 (C) | 0.0 (C) |
| | 6 | DJ0001-5/amb-2 | DNM | DNM | DNM | 103.0 | DNM | DNM |
| | 9 | DJ0001-5/amb-1 | Clear | Colorless | 4.53 | 103.9 | 2.6 (C) | 0.1 (C) |
| | 9 | DJ0001-5/amb-2 | DNM | DNM | DNM | 103.3 | DNM | DNM |
| | 12 | DJ0001-5/amb-1 | Clear | Colorless | 4.52 | 104.3 | 0.3 (C) | 0.0 (C) |
| | 12 | DJ0001-5/amb-2 | DNM | DNM | DNM | 103.5 | DNM | DNM |
| | 14 | DJ0001-5/amb-1 | DNM | DNM | DNM | DNM | DNM | DNM |
| | 14 | DJ0001-5/amb-2 | DNM | DNM | DNM | DNM | DNM | DNM |

(continued)

| Storage Conditions | Time (months) | Sample No. | Clarity | Color | pH | Pazopanib Content (% label claim) | Particle Count ≥ 10 µm | Particle Count ≥ 25 µm |
|---|---|---|---|---|---|---|---|---|
| **25°C/60% RH (packaged in blow-filled seal container)** | 1 | DJ0001-25/60-1 | Clear | Colorless | 4.57 | 103.1 | 1.1 (C) | 0.1 (C) |
| | 1 | DJ0001-25/60-2 | DNM | DNM | DNM | 102.8 | DNM | DNM |
| | 3 | DJ0001-25/60-1 | Clear | Colorless | 4.50 | 104.4 | 4.1 (C) | 0.1 (C) |
| | 3 | DJ0001-25/60-2 | DNM | DNM | DNM | 104.3 | DNM | DNM |
| | 6 | DJ0001-25/60-1 | Clear | Colorless | 4.48 | 103.9 | 0.7 (C) | 0.0 (C) |
| | 6 | DJ0001-25/60-2 | DNM | DNM | DNM | 104.0 | DNM | DNM |
| | 9 | DJ0001-25/60-1 | Clear | Colorless | 4.56 | 104.7 | 1.6 (C) | 0.0 (C) |
| | 9 | DJ0001-25/60-2 | DNM | DNM | DNM | 108.0 | DNM | DNM |
| | 12 | DJ0001-25/60-1 | Clear | Colorless | 4.50 | 106.2 | 4.0 (C) | 0.3 (C) |
| | 12 | DJ0001-25/60-2 | DNM | DNM | DNM | 104.7 | DNM | DNM |
| | 14 | DJ0001-25/60-1 | DNM | DNM | DNM | DNM | DNM | DNM |
| | 14 | DJ0001-25/60-2 | DNM | DNM | DNM | DNM | DNM | DNM |

(continued)

| Storage Conditions | Time (months) | Sample No. | Clarity | Color | pH | Pazopanib Content (% label claim) | Particle Count ≥ 10 μm | Particle Count ≥ 25 μm |
|---|---|---|---|---|---|---|---|---|
| **40°C/25% RH (packaged in blow-filled seal container)** | 1 | DJ0001-40/25-1 | Clear | Colorless | 4.60 | 104.4 | 0.3 (A) | 0.0 (A) |
| | 1 | DJ0001-40/25-2 | DNM | DNM | DNM | 103.8 | DNM | DNM |
| | 3 | DJ0001-40/25-1 | Clear | Colorless | 4.50 | 105.3 | 0.2 (B) | 0.0 (B) |
| | 3 | DJ0001-40/25-2 | DNM | DNM | DNM | 105.1 | DNM | DNM |
| | 6 | DJ0001-40/25-1 | Clear | Colorless | 4.51 | 104.1 | 0.3 (C) | 0.0 (C) |
| | 6 | DJ0001-40/25-2 | DNM | DNM | DNM | 104.8 | DNM | DNM |
| | 9 | DJ0001-40/25-1 | Clear | Colorless | 4.55 | 107.5 | 0.6 (C) | 0.1 (C) |
| | 9 | DJ0001-40/25-2 | DNM | DNM | DNM | 105.1 | DNM | DNM |
| | 12 | DJ0001-40/25-1 | DNM | DNM | DNM | DNM | DNM | DNM |
| | 12 | DJ0001-40/25-2 | DNM | DNM | DNM | DNM | DNM | DNM |
| | 14 | DJ0001-40/25-1 | Clear | Colorless | 4.50 | 105.8 | 0.3 (C) | 0.0 (C) |
| | 14 | DJ0001-40/25-2 | DNM | DNM | DNM | 107.1 | DNM | DNM |

**Example 4: Determination of Binding Constant $K_b$ for Pazopanib**

**Methods**

The binding constant between a drug substance and complexing agent can be determined using spectroscopic techniques as outlined by Connors and references therein (Kenneth A. Connors: "Binding Constants", Wiley-Interscience; 1. edition (April 1987)).

The simplified equilibrium for complexation between pazopanib (034 in Equation 1 shown below) drug substance and β-cyclodextrin sulfobutylether (e.g., Captisol® from Cydex Pharmaceuticals, Inc., Lenexa, KS) has been shown as Equation 1, where $K_d$ is the dissociation constant and $K_b$ is the binding constant, $034_{(s)}$ is solid precipitate for pazopanib, $034_{(aq)}$ is the aqueous concentration of unbound pazopanib (free state without complexation), $Captisol_{(aq)}$ is the difference between the initial Captisol concentration and the concentration of Captisol in the complexed state with drug substance and pazopanib. $Captisol_{(aq)}$ is the concentration of the complex between pazopanib and 034•Captisol (complexed state).

Equation 1

$$\downarrow 034_{(s)} \rightleftharpoons 034_{(aq)} + Captisol_{(aq)} \rightleftharpoons 034 \cdot Captisol_{(aq)}$$

$$K_d = \frac{[034_{(aq)}] x [Captisol_{(aq)}]}{[034 \cdot Captisol_{(aq)}]} \qquad K_b = \frac{1}{K_d}$$

A titration experiment is performed using pazopanib (approximately 0.05 mg/ml, 10-4M) solution by varying the concentration of β-cyclodextrin sulfobutylether solution and measuring the fluorescence intensity (excitation at 342 nm, emission at 375 nm) of the resulting solutions. Initial Captisol concentration are chosen to describe the entire titration curve appropriately and to prevent nucleation and precipitation of pazopanib in the solution. Typically initial Captisol concentrations range from not more than approximately 0.05 mg/ml to at least approximately 3.5 mg/ml.

The measured Fluorescence intensity is directly related to the concentration of the complexed state 034·Captisol(aq) since uncomplexed pazopanib 034(aq) and Captisol Captisol(aq) have negligible fluorescence yield. The measured fluorescence intensities are fitted using a non-linear simplex fitting routine to determine the value of the binding constant $K_b$. The Fluorescence intensities are plotted as a function of β-cyclodextrin sulfobutylether concentration according to Benesi and Hildebrandt to determine the binding stoichiometry. These determinations are performed at specific values of pH and temperature of the solutions.

Instrumentation:

Varian Cary Eclipse Fluorescence spectrometer (SN: EL05043801) with a 1cm cuvette and orthogonal detection geometry or equivalent. The temperature of the solution is controlled by controlling the temperature of the cuvette holder and ensuring good contact.

**Results**

The fluorescence intensities as a function of β-cyclodextrin sulfobutylether concentration at a pH of 4 and temperature of 40°C are shown in Figure 1 and yield a binding constant, $K_b$, of 9763 $mol^{-1}$.

The fluorescence intensities as a function of β-cyclodextrin sulfobutylether concentration at a pH of 5 and temperature of 25°C are shown in Figure 2 and yield a binding constant, $K_b$, of 9130 $mol^{-1}$.

**Example 5: Determination of Solubility of Pazopanib as a Function of pH**

**Methods**

1) Prep 25 mM phosphate buffer (using monobasic Sodium Phosphate, monohydrate), adjust the buffer solution to

different target pH using 1N NaOH/HCl.
2) Add excessive pazopanib free base to the above solutions (~20 mg free base per 10 mL buffer solution), vortex the solutions, and mix well. Measure the pH of the solubility solutions, adjust the pH to target value if needed.
3) Place the solubility solutions into a chamber with a constant temperature of 5 °C. Equilibrate the solutions for 5 days with agitation.
4) After 5 days, measure the pH of the solubility solution immediately, and record the pH, and filter the solution into a test tube using a 0.22um PVDF filter.
5) Dilute the solution by 1:1 (v/v) with 50:50:0.1 water:acetonitrile:TFA (v/v/v) diluent, and mix well.
6) The solubility of the solutions is analyzed by HPLC at the detection wavelength of 268 nm.

HPLC parameters:

Mobile Phase A: 100:0.1 (v/v) Water:TFA

Mobile Phase B: 100:0.1 (v/v) Acetonitrile:TFA

Column: Phenomenex Develosil RPAqueous-3, C-30, 3 um, 150 mm x 4.6 mm

Column temperature: 35°C

Flow rate: Isocratic at 1.0 mL/min, A/B = 60/40

Detector wavelength: 268 nm

Injection volume: 10 μ L

Total run time: 4 minutes

**Results**

Solubility at 25°C was determined as follows using a procedure the same as or similar to the foregoing: 0.000006 mg/mL (1.37 x $10^{-8}$ mol/L) at pH 5.0, 0.000025 mg/mL (5.71 x $10^{-8}$ mol/L) at pH 4.5; 0.000534 mg/mL (1.22 x $10^{-6}$ mol/L) at pH 4.25; 0.001043 mg/mL (2.38 x $10^{-6}$ at pH 4.0; and 0.02 mg/mL (4.57 x $10^{-5}$ mol/mL) at pH 3.5.

**Example 6: Calculation of $U_{CD}$**

$$U_{CD} = (KS_o/1+KS_o)(m_{CD}/m_D)(MW_D/MW_{CD})$$

Dose of drug, $m_D$
Dose of cyclodextrin, $m_{CD}$
Molecular weight drug, MW $_D$
Molecular weight cyclodextrin, MW $_{CD}$
Binding constant, K, as described in Example 4 above.
Solubility, $S_o$, as described in Example 5 above.
from Rao, V.M., Stella, V.J., J Pharm Sci, 92, 5 927, May 2003.

The UCD calculation for a 10 mg/mL pazopanib and 9% Captisol® (β-cyclodextrin sulfobutylether) solution at various pH levels is given below in Table 7.

**Table 7**

| | pH 3.5 | pH 4.0 | pH 4.25 | pH 4.5 | pH 5.0 |
|---|---|---|---|---|---|
| Dose of drug, $m_D$ (mg) | 0.4 | 0.4 | 0.4 | 0.4 | 0.4 |

(continued)

| | pH 3.5 | pH 4.0 | pH 4.25 | pH 4.5 | pH 5.0 |
|---|---|---|---|---|---|
| Dose of cyclodextrin, as β-cyclodextrin sulfobutvlether, $m_{CD}$ (mg) | 3.6 | 3.6 | 3.6 | 3.6 | 3.6 |
| MW $_D$ | 437.5 | 437.5 | 437.5 | 437.5 | 437.5 |
| *MW* $_{CD}$ | 2200 | 2200 | 2200 | 2200 | 2200 |
| $S_o$ (mol/L) | $4.57 \times 10^{-5}$ | $2.38 \times 10^{-6}$ | $1.22 \times 10^{-6}$ | $5.71 \times 10^{-8}$ | $1.37 \times 10^{-8}$ |
| K (L/mol)* | 10000 | 10000 | 10000 | 10000 | 10000 |
| $(KS_o)/(1+KS_o)$ | 0.314 | 0.023 | 0.012 | 0.0006 | 0.0001 |
| $[m_{CD}/ m_D]$ | 9 | 9 | 9 | 9 | 9 |
| $[MW_D/MW_{CD}]$ | 0.199 | 0.199 | 0.199 | 0.199 | 0.199 |
| $U_{CD}$ | 0.56 | 0.042 | 0.022 | 0.001 | 0.0002 |
| * A conservative value of 10,000 was used for these calculations. A lower value of K, such as those determined in Example 4 above, would result in an even lower $U_{CD}$ value. | | | | | |

The UCD calculation for a 10 mg/mLpazopanib and 13% Captisol® (β-cyclodextrin sulfobutylether) solution at various pH levels is given below in Table 8.

**Table 8**

| | pH 3.5 | pH 4.0 | pH 4.25 | pH 4.5 | pH 5.0 |
|---|---|---|---|---|---|
| Dose of drug, $m_D$ (mg) | 0.4 | 0.4 | 0.4 | 0.4 | 0.4 |
| Dose of cyclodextrin, as β-cyclodextrin sulfobutylether, $m_{CD}$ (mg) | 4.8 | 4.8 | 4.8 | 4.8 | 4.8 |
| MW $_D$ | 437.5 | 437.5 | 437.5 | 437.5 | 437.5 |
| MW $_{CD}$ | 2200 | 2200 | 2200 | 2200 | 2200 |
| $S_o$ (mol/L) | $4.57 \times 10^{-5}$ | $2.38 \times 10^{-6}$ | $1.22 \times 10^{-6}$ | $5.71 \times 10^{-8}$ | $1.37 \times 10^{-8}$ |
| $K_o$ (L/mol)* | 10000 | 10000 | 10000 | 10000 | 10000 |
| $(KS_o)/(1+KS_o)$ | 0.314 | 0.023 | 0.012 | 0.0006 | 0.0001 |
| $[m_{CD}/m_D]$ | 13 | 13 | 13 | 13 | 13 |
| $[MW_D/MW_{CD}]$ | 0.199 | 0.199 | 0.199 | 0.199 | 0.199 |
| $U_{CD}$ | 0.81 | 0.060 | 0.031 | 0.0015 | 0.0004 |
| * A conservative value of 10,000 was used for these calculations. A lower value of K, such as those determined in Example 4 above, would result in an even lower $U_{CD}$ value. | | | | | |

The UCD calculation for a 10 mg/mL pazopanib and 2% Captisol® (β-cyclodextrin sulfobutylether) solution at various pH levels is given below in Table 9.

**Table 9**

| | pH 3.5 | pH 4.0 | pH 4.25 | pH 4.5 | pH 5.0 |
|---|---|---|---|---|---|
| Dose of drug, $m_D$ (mg) | 0.4 | 0.4 | 0.4 | 0.4 | 0.4 |
| Dose of cyclodextrin, as β-cyclodextrin sulfobutylether, $m_{CD}$ (mg) | 0.8 | 0.8 | 0.8 | 0.8 | 0.8 |
| MW $_D$ | 437.5 | 437.5 | 437.5 | 437.5 | 437.5 |
| MW $_{CD}$ | 2200 | 2200 | 2200 | 2200 | 2200 |

(continued)

| | pH 3.5 | pH 4.0 | pH 4.25 | pH 4.5 | pH 5.0 |
|---|---|---|---|---|---|
| $S_o$ (mol/L) | 4.57 x $10^{-5}$ | 2.38 x $10^{-6}$ | 1.22 x $10^{-6}$ | 5.71 x $10^{-8}$ | 1.37 x $10^{-8}$ |
| $K_o$ (L/mol)* | 10000 | 10000 | 10000 | 10000 | 10000 |
| $(KS_o)/(1+KS_o)$ | 0.314 | 0.023 | 0.012 | 0.0006 | 0.0001 |
| $[m_{CD}/ m_D]$ | 2 | 2 | 2 | 2 | 2 |
| $[MW_D/MW_{CD}]$ | 0.199 | 0.199 | 0.199 | 0.199 | 0.199 |
| $U_{CD}$ | 0.12 | 0.0093 | 0.0048 | 0.0002 | 0.0001 |
| * A conservative value of 10,000 was used for these calculations. A lower value of K, such as those determined in Example 4 above, would result in an even lower $U_{CD}$ value. | | | | | |

As described in the Background with reference to the Rao article, the $U_{CD}$ allows the formulator to determine if the use of cyclodextrins in the formulation of poorly water-soluble drugs has the potential to provide a significant solubilization advantage. When the $U_{CD}$ dimensionless number is less than 1, the complexation alone is not enough for complete solubilization. In view of the low $U_{CD}$ values for the 10 mg/mL compositions, it would have been unexpected that compositions such as these would have exhibited the stability necessary for use as clinical trial formulations.

**Example 7 Measurement of pazopanib p$K_{a2}$ by potentiometric titration**

**Materials**

Captisol® (β-cyclodextrin suflobutyl ether) was obtained from Cydex Pharmaceuticals, Inc., Lenexa, KS. Pazopanib HCl GlaxoSmithKline, Pennsylvania, PA).Sodium hydroxide (NaOH) titration standards were prepared from Titrisol® 0.1 mol/L standards and sodium chloride (NaCl, batch number K40817904012) were obtained from Merck Chemicals (Darmstadt, Germany). Benzoic acid thermochemical standard tablets were from BDH Limited (Poole, England). Benzoic acid ACS reagent and phenylalanine methyl ester HCl were from Sigma-Aldrich (St Louis, USA). All solids were stored in a desiccator. Calibration pH standards were purchased in single use sachets from Metrohm AG (Zofigen, Switzerland). Argon gas (Grade 5.0) was obtained from BOC gases (North Ryde, NSW, Australia).

**Equipment**

A Metrohm AG 907 Titrando™ potenitometric autotitrator system was used for all potentiometric titrations. The Titrando™ was fitted with an 800 Dosino™ dosing unit and an iUnitrode pH electrode (very low sodium response) filled with 3 mol/L KCl internal electrolyte. The system was controlled by Tiamo™ light version 2.2 autotitration software.

All titrations and pH electrode calibrations were carried out in double walled glass thermostatted titration vessels (maximum volume, 90 mL) obtained from Metrohm AG. The titration vessels were externally thermostatted to measurement temperature using a Heto CBN 8-30 water bath fitted with a Heto HMT 200 thermostat pump. All connecting hoses were insulated to minimized heat transfer to or from the surroundings. The contents of the titration vessel were stirred with a Metrohm AG 802 propeller stirrer.

**Methods**

**Preparation of titration standards for p$K_a$ determination**

The careful preparation of 0.1000N NaOH standard titrant is critical for accurate p$K_a$ determinations by potentiometric titration. Further, elimination of carbon dioxide ($CO_2$) from the NaOH standard is critical for titration accuracy. Therefore, titration standards must be prepared with carbonate free water under argon, and stored under argon.

To prepare the carbonate-free water, a 1 L glass bottle was filled with 900 mL of MilliQ® water and was boiled while stirring for 30 mins. The headspace of the bottle was filled with argon gas before the water was allowed to cool with the lid on loosely. Once cooled, the headspace was filled with argon and the lid was screwed on tightly.

When the temperature of the de-carbonated water was close to room temperature (around 25°C or less), NaOH titration standard was prepared. Under an argon blanket, the plastic ampoule containing the Titrisol® solution was inserted in the neck of a 1 L volumetric flask and the solution was dispensed according to the manufacturer's instructions. De-carbonated water was used to rinse the remaining NaOH solution from the ampoule. When the temperature of both NaOH solution and the de-carbonated water reached 20°C, the NaOH solution was made up to volume with the de-carbonated water and then mixed thoroughly. The headspace of the bottle was filled with argon before being connecting it to the Dosino titration dosing unit and the dosing unit was fitted with a soda lime guard tube. Any unused NaOH solution was stored under argon to prevent absorption of $CO_2$ by the system.

**Calibration of the pH electrode**

The calibration procedure was carried out prior to every titration, and was conducted at the temperature at which the titration experiment was to be carried out. Sample solutions were also equilibrated to measurement temperature prior to the commencement of the titration and during the titration.

A sachet of each calibration standard solution (nominally pH 4 and pH 7) was dispensed into separate, clean, dry thermostatted titration vessels. The standard solutions were allowed time to equilibrate to the experimental temperature. Calibration commenced by firstly transferring the vessel containing the pH 4 standard solution to the autotitrator. A slow stream of argon gas was allowed through the gas inlet into the titration vessel. Using the Tiamo™ software, the stirrer was set at a rate of 1 (slow stirring, arbitrary). After the pH reading had stabilized (automatically detected by the software), the pH 4 solution was removed and the electrode was rinsed with MilliQ® water before being carefully dried with a Kimwipe® tissue. These steps were then repeated using the pH 7 calibration standard. The calibration was cross-checked by measuring the pH of the pH 4 reference standard in 'measurement' mode. Prior to the commencement of any titrations, the volume of standard in the Dosino™ was checked to ensure that there was adequate 0.1000N NaOH standard to complete the titration. For details of NaOH standard preparation see Section 0.

**Validation of p$K_a$ determinations by potentiometric titration using reference compounds**

Benzoic acid (p$K_a$ = 4.20) (primary standard) and phenylalanine methyl ester hydrochloride (p$K_a$ = 7.11) (secondary standard) are compounds for which the p$K_a$ values are accurately reported in the literature.[1] Determination of the p$K_a$ values of these two reference compounds using the Titrando® autotitration system, to an accuracy of $\pm$ 0.03, served as suitable validation for the potentiometric determination of pazopanib p$K_{a2}$ in the presence of Captisol®. It should be noted that all test solutions should be equilibrated to the experimental temperature prior to commencing any titration. Temperatures were measured in the titration vessel with one of a matched pair of reference mercury-in-glass thermometers, with correction for the emergent stem.

**Preparation of benzoic acid standard**

Benzoic acid (thermochemical standard) tablets were crushed using an agate mortar and pestle. The benzoic acid powder was then transferred to a shell vial. The crushed powder was stored overnight in a desiccator with activated silica gel. Approximately 61.5 mg of the powdered benzoic acid was weighed into a clean glass beaker. The powder was tipped carefully into the thermostatted glass titration vessel and the beaker was reweighed to calculate the mass transferred to the thermostatted titration vessel. The mass of material dispensed into the titration vessel was recorded in the Tiamo™ software. Two drops of ethanol were added to the benzoic acid powder to aid dissolution. Using an A grade 50 mL volumetric glass pipette, 50.0 mL of MilliQ® water was dispensed into the titration vessel. The content of the vessel was stirred until the benzoic acid had completely dissolved.

Prior to potentiometric titration, the pH electrode was calibrated as described above. When the benzoic acid solution had equilibrated to the measurement temperature, the vessel containing benzoic acid solution was positioned on the autotitrator. The Tiamo software was set at a stirring rate of 1 (slow stir rate, arbitrary units), with 60 secs between NaOH aliquot additions. The end point of the titration was set at pH 6. Prior to subsequent titrations, the pH electrode was re-calibrated as described above.

**Preparation of phenylalanine methyl ester HCl standard**

Approximately 107 mg of phenylalanine methyl ester HCl was weighed into a clean glass beaker. The powder was tipped carefully into the thermostatted glass titration vessel and the beaker was reweighed to calculate the mass transferred to the titration vessel. The mass of material dispensed into the titration vessel was recorded in the Tiamo™ software.

Using a 50 mL glass volumetric pipette, 50.0 mL of MilliQ® water was dispensed into the titration vessel. The content of the vessel was stirred until the phenylalanine methyl ester HCl had dissolved.

Prior to potentiometric titration, the pH electrode was then calibrated according to Section 0. When the phenylalanine methyl ester HCl solution had equilibrated to the measurement temperature, the vessel containing phenylalanine

methyl ester HCl was transferred to the autotitrator and a slow argon gas stream was allowed to flow through the gas inlet of the titration vessel. The Tiamo™ software was set at a stirring rate of 1 (slow) with 60 secs between NaOH aliquot additions. The end point of the titration was set at pH = 9.

Prior to subsequent titrations, the pH electrode was re-calibrated following the method outlined in Section 0

**Measurement of pazopanib p$K_{a2}$ by potentiometric titration**

**Preparation of Captisol® solutions containing pazopanib**

A 70 mg/mL Captisol solution was prepared by weighing out nominally 70 g of Captisol® in a clean and dry 1 L volumetric flask. MilliQ® water was added to the flask to 2/3 of the total volume. Captisol® was dissolved by agitating the flask, before making up to the final volume with MilliQ® water. The water content (7-8% w/w) of the Captisol®, as determined by Karl Fischer titration (separate protocol), was taken into account when weighing the Captisol® powder in the preparation of all Captisol® solutions.

In a clean and dry 250 mL volumetric flask, approximately 1.354 g of pazopanib HCl was weighed (the actual mass of pazopanib hydrochloride was recorded in order to calculate final pazopanib hydrochloride concentration). The 70 mg/mL Captisol® solution was added to the pazopanib HCl, filling to approximately 2/3 of the total volume of the flask. Pazopanib HCl was then dissolved by sonication, checking carefully for any undissolved particles. Subsequently, 365.25 mg of NaCl was added and agitated until dissolved. The solution was made up to volume and stored in the refrigerator if not for immediate use. The target final pazopanib HCl concentration of the solution was 5.417 mg/mL and the target final NaCl concentration of the solution was 1.461 mg/mL.

**Determination of pazopanib p$K_{a2}$ in the presence of Captisol®**

Add to a separate jacketed vessel held at the same temperature as was used for the pH calibration. Using a 20.00 mL and a 25.00 mL A grade glass pipette, dispense 45.0 mL of the 70 mg/mL Captisol®-pazopanib HCl solution (prepared as described in Section 0) into a thermostatted glass titration vessel, which had been equilibrated to the measurement temperature. pH electrode calibration was performed while the Captisol®-pazopanib HCl solution was equilibrating.

The titrations were carried out using the Tiamo software with the stirring rate set at 1 (slow) and the wait time between aliquots set to 60 seconds. The end point of the titration was pH = 7.5. Prior to subsequent titrations, the pH electrode was re-calibrated following the method outlined in Section 0.

Potentiometric titrations of Captisol®-pazopanib HCl solutions were carried out at 5, 10, 15, 20, 25, 30 and 35°C. Measurements were repeated in triplicate or until p$K_{a2}$ result reproducibility was within ± 0.03.

**Data analysis and calculation of pazopanib p$K_{a2}$**

Analysis of the potentiometric titration data (pH value as a function of NaOH aliquot added) used the Henderson-Hasselbalch equation with full corrections for hydronium ion concentration, hydroxyl ion concentration and mean ionic activity coefficient, as outlined by Albert and Serjeant[2] and further discussed elsewhere:

$$pKa = pH + \left\{\frac{[Y] - [H^+] - [Na^+] + [OH^-]}{[H^+] + [Na^+] - [OH^-]}\right\} - log\gamma_\pm$$

The mean ionic activity coefficient was calculated using the full Debye-Hückel equation:

$$-log\gamma_\pm = A|z_+z_-|\frac{\sqrt{I}}{1 + Ba_o\sqrt{I}}$$

where I is the ionic strength, A and B are tabulated constants whose values depend only on temperature and dielectric constant, and $a_o$ is the ion size parameter, which was set at 3 Å units. As described in Ref. 1, the interdependent calculations of $[H^+]$ and ionic strength (I) were iterated five times to ensure that convergence was achieved; this usually occurred after the 3rd iteration. The calculations were performed with MS Excel®.

## REFERENCES

1. Prankerd, R.J., Critical compilation of Ionisation Constants. In: Brittain, H.B. Ed. Profiles of drug substances, exipients and related methodology. San Diego: Academic Press-Elsevier; Vol 33, 2007.
2. Albert AA, Serjeant EP, The Determination of Ionisation Constants, 3rd Ed., Chapman and Hall, London UK (1984), Chs. 2 and 3)

## Claims

**1.** A pharmaceutical composition comprising:

about 10 mg pazopanib/mL of the composition;
about 2.0 to about 13.0 % w/w of a modified cyclodextrin;
a pH adjusting agent as needed to provide a pH of 3.5 to 5.7;
a tonicity adjusting agent as needed to provide an osmolality of 200 to 400 mOsm; and
water.

**2.** A pharmaceutical composition according to claim 1 comprising:

about 10 mg pazopanib/mL of the composition;
from about 2.0 to about 13.0 % w/w of a modified cyclodextrin, said modified cyclodextrin being selected such that the modified cyclodextrin results in the $pK_a$ of pazopanib with said modified cyclodextrin in water being lower than the $pK_a$ of pazopanib alone in water;
a pH adjusting agent as needed to provide a pH of 3.5 to 5.7;
a tonicity adjusting agent as needed to provide an osmolality of 200 to 400 mOsm; and
water;
wherein the composition is stable for at least 2 months.

**3.** The pharmaceutical composition according to claim 2, wherein the modified cyclodextrin is selected such that the modified cyclodextrin results in the $pK_a$ of pazopanib with said modified cyclodextrin in water being at least 0.4 lower than the $pK_a$ of pazopanib alone in water in a 10 mg pazopanib/mL water solution.

**4.** The pharmaceutical composition according to any one of claims 2 or 3, wherein the modified cyclodextrin is selected such that the modified cyclodextrin results in the $pK_a$ of pazopanib with said modified cyclodextrin in water being at least 0.8 lower than the $pK_a$ of pazopanib alone in water.

**5.** A pharmaceutical composition according to claim 1 comprising:

about 10 mg pazopanib/mL of the composition;
about 2.0 to about 13.0% w/w of a modified cyclodextrin; and
a pH adjusting agent as needed to provide a pH of 3.5 to 5.7;
a tonicity adjusting agent as needed to provide an osmolality of 200 to 400 mOsm; and
water;
wherein the composition has a $U_{CD}$ value in the range of 0.0002 to 0.6 at a temperature of 25°C, and wherein the composition is stable for at least 2 months.

**6.** A pharmaceutical composition according to claim 1 comprising:

about 10 mg pazopanib/mL of the composition;
about 2.0 to about 13.0% w/w of a modified cyclodextrin; and
a pH adjusting agent as needed to provide a pH of 3.5 to 5.7;
a tonicity adjusting agent as needed to provide an osmolality of 200 to 400 mOsm; and
water;
wherein the composition is a super-saturated aqueous solution of pazopanib, and wherein the composition is stable for at least 2 months.

**7.** The pharmaceutical composition according to any of the preceding claims, wherein the composition has a pH of from about 4 to about 4.5.

**8.** The pharmaceutical composition according to claims 1 to 6, wherein the osmolality of the composition is from about 270 to about 330 mOsm.

**9.** The pharmaceutical composition according to claims 1 to 6, wherein the modified cyclodextrin is selected from the group consisting of hydroxypropyl-β- cyclodextrin, methyl-β-cyclodextrin, β-cyclodextrin sulfobutylether and combinations thereof

**10.** The pharmaceutical composition according to claim 9, wherein the modified cyclodextrin is β-cyclodextrin sulfobutylether.

**11.** The pharmaceutical composition according to claims 9 and 10, wherein the amount of the modified cyclodextrin is in the range of about 6.0 % to about 10.0 % w/w.

**12.** The pharmaceutical composition of any one of the preceding claims, wherein the modified cyclodextrin is suitable for administration to the eye of a human.

**13.** The pharmaceutical composition of any one of the preceding claims, wherein the composition is an eye drop formulation suitable for administration to a human.

**14.** The pharmaceutical composition according to any one of the preceding claims, wherein the composition is stable for at least 6 months.

**15.** The pharmaceutical composition according to any one of the preceding claims, wherein the composition is stable for at least 12 months.

**16.** The pharmaceutical composition according to any one of the preceding claims, further comprising a buffering agent.

**17.** The pharmaceutical composition according to claim 15, wherein said buffering agent is a phosphate buffering agent.

**18.** The pharmaceutical composition of any one of the preceding claims, wherein the pH adjusting agent is selected from the group consisting of sodium hydroxide, hydrochloric acid and combinations thereof.

**19.** A pharmaceutical composition according to claim 1 comprising:
about 10 mg pazopanib/mL of the composition;
about 9 % β-cyclodextrin sulfobutylether;
a pH adjusting agent as needed to provide a pH of 3.5 to 5.7;
a tonicity adjusting agent as needed to provide an osmolality of 200 to 400 mOsm; and water.

**20.** The pharmaceutical composition of claim 19, wherein the composition is an eye drop formulation suitable for administration to a human.

**21.** A method of preparation of a super-saturated solution of pazopanib, said method comprising:

forming an aqueous solution of an acid addition salt of pazopanib and a modified cyclodextrin suitable for use in an ophthalmic formulation; and
adjusting the pH of said solution to between 3.5 to 5.7 to obtain a super-saturated solution of pazopanib, wherein the concentration of the acid addition salt of pazopanib solubilized in the super-saturated solution is equivalent to about 10 mg/ml of pazopanib.

**22.** The method according to claim 21, wherein the acid addition salt of pazopanib is pazopanib hydrochloride.

**23.** The method according to claim 21 or 22, wherein the modified cyclodextrin is selected from the group consisting of hydroxypropyl-β- cyclodextrin, methyl-β-cyclodextrin, β-cyclodextrin sulfobutylether and combinations thereof.

**Patentansprüche**

1. Pharmazeutische Zusammensetzung, umfassend:

   etwa 10 mg Pazopanib/ml der Zusammensetzung;
   etwa 2,0 bis etwa 13,0 % G/G eines modifizierten Cyclodextrins; und
   ein pH-Einstellmittel, wie zur Bereitstellung eines pH-Wertes von 3,5 bis 5,7 erforderlich;
   ein Tonizitäts-Einstellmittel, wie zur Bereitstellung einer Osmolalität von 200 bis 400 mOsm erforderlich; und
   Wasser.

2. Pharmazeutische Zusammensetzung gemäss Anspruch 1, umfassend:

   etwa 10 mg Pazopanib/ml der Zusammensetzung;
   etwa 2,0 bis etwa 13,0 % G/G eines modifizierten Cyclodextrins, wobei das modifizierte Cyclodextrin so ausgewählt ist, dass das modifizierte Cyclodextrin in einem $pK_a$ von Pazopanib mit dem modifizierten Cyclodextrin in Wasser resultiert, der niedriger als der $pK_a$ von Pazopanib alleine in Wasser ist;
   ein pH-Einstellmittel, wie zur Bereitstellung eines pH-Wertes von 3,5 bis 5,7 erforderlich;
   ein Tonizitäts-Einstellmittel, wie zur Bereitstellung einer Osmolalität von 200 bis 400 mOsm erforderlich; und
   Wasser;
   wobei die Zusammensetzung für mindestens 2 Monate stabil ist.

3. Pharmazeutische Zusammensetzung gemäss Anspruch 2, wobei das modifizierte Cyclodextrin so ausgewählt ist, dass das modifizierte Cyclodextrin in einem $pK_a$ von Pazopanib mit dem modifizierten Cyclodextrin in Wasser resultiert, der mindestens 0,4 niedriger als der $pK_a$ von Pazopanib alleine in Wasser ist, in einer Lösung aus 10 mg Pazopanib/ml Wasser.

4. Pharmazeutische Zusammensetzung gemäss irgendeinem der Ansprüche 2 oder 3, wobei das modifizierte Cyclodextrin so ausgewählt ist, dass das modifizierte Cyclodextrin in einem $pK_a$ von Pazopanib mit dem modifizierten Cyclodextrin in Wasser resultiert, der mindestens 0,8 niedriger als der $pK_a$ von Pazopanib alleine in Wasser ist.

5. Pharmazeutische Zusammensetzung gemäss Anspruch 1, umfassend:

   etwa 10 mg Pazopanib/ml der Zusammensetzung;
   etwa 2,0 bis etwa 13,0 % G/G eines modifizierten Cyclodextrins; und
   ein pH-Einstellmittel, wie zur Bereitstellung eines pH-Wertes von 3,5 bis 5,7 erforderlich;
   ein Tonizitäts-Einstellmittel, wie zur Bereitstellung einer Osmolalität von 200 bis 400 mOsm erforderlich; und
   Wasser;
   wobei die Zusammensetzung einen $U_{CD}$-Wert im Bereich von 0,0002 bis 0,6 bei einer Temperatur von 25°C aufweist und wobei die Zusammensetzung für mindestens 2 Monate stabil ist.

6. Pharmazeutische Zusammensetzung gemäss Anspruch 1, umfassend:

   etwa 10 mg Pazopanib/ml der Zusammensetzung;
   etwa 2,0 bis etwa 13,0 % G/G eines modifizierten Cyclodextrins; und
   ein pH-Einstellmittel, wie zur Bereitstellung eines pH-Wertes von 3,5 bis 5,7 erforderlich;
   ein Tonizitäts-Einstellmittel, wie zur Bereitstellung einer Osmolalität von 200 bis 400 mOsm erforderlich; und
   Wasser;
   wobei die Zusammensetzung eine übersättigte wässrige Lösung von Pazopanib ist und wobei die Zusammensetzung für mindestens 2 Monate stabil ist.

7. Pharmazeutische Zusammensetzung gemäss irgendeinem der vorhergehenden Ansprüche, wobei die Zusammensetzung einen pH-Wert von etwa 4 bis etwa 4,5 aufweist.

8. Pharmazeutische Zusammensetzung gemäss Ansprüchen 1 bis 6, wobei die Osmolalität der Zusammensetzung

etwa 270 bis etwa 330 mOsm beträgt.

**9.** Pharmazeutische Zusammensetzung gemäss Ansprüchen 1 bis 6, wobei das modifizierte Cyclodextrin aus der Gruppe bestehend aus Hydroxypropyl-β-cyclodextrin, Methyl-β-cyclodextrin, β-Cyclodextrinsulfobutylether und Kombinationen davon ausgewählt ist.

**10.** Pharmazeutische Zusammensetzung gemäss Anspruch 9, wobei das modifizierte Cyclodextrin β-Cyclodextrinsulfobutylether ist.

**11.** Pharmazeutische Zusammensetzung gemäss Anspruch 9 und 10, wobei die Menge des modifizierten Cyclodextrins im Bereich von etwa 6,0 bis etwa 10,0 % G/G liegt.

**12.** Pharmazeutische Zusammensetzung gemäss irgendeinem der vorhergehenden Ansprüche, wobei das modifizierte Cyclodextrin zur Verabreichung an das Auge eines Menschen geeignet ist.

**13.** Pharmazeutische Zusammensetzung gemäss irgendeinem der vorhergehenden Ansprüche, wobei die Zusammensetzung eine Augentropfenformulierung ist, die zur Verabreichung an einen Menschen geeignet ist.

**14.** Pharmazeutische Zusammensetzung gemäss irgendeinem der vorhergehenden Ansprüche, wobei die Zusammensetzung für mindestens 6 Monate stabil ist.

**15.** Pharmazeutische Zusammensetzung gemäss irgendeinem der vorhergehenden Ansprüche, wobei die Zusammensetzung für mindestens 12 Monate stabil ist.

**16.** Pharmazeutische Zusammensetzung gemäss irgendeinem der vorhergehenden Ansprüche, die ferner ein Puffermittel umfasst.

**17.** Pharmazeutische Zusammensetzung gemäss Anspruch 15, wobei das Puffermittel ein Phosphatpuffer ist.

**18.** Pharmazeutische Zusammensetzung gemäss irgendeinem der vorhergehenden Ansprüche, wobei das pH-Einstellmittel aus der Gruppe bestehend aus Natriumhydroxid, Salzsäure und Kombinationen davon ausgewählt ist.

**19.** Pharmazeutische Zusammensetzung gemäss Anspruch 1, umfassend:

etwa 10 mg Pazopanib/ml der Zusammensetzung;
etwa 9 % β-Cyclodextrinsulfobutylether;
ein pH-Einstellmittel, wie zur Bereitstellung eines pH-Wertes von 3,5 bis 5,7 erforderlich;
ein Tonizitäts-Einstellmittel, wie zur Bereitstellung einer Osmolalität von 200 bis 400 mOsm erforderlich; und
Wasser.

**20.** Pharmazeutische Zusammensetzung gemäss Anspruch 19, wobei die Zusammensetzung eine Augentropfenformulierung ist, die zur Verabreichung an einen Menschen geeignet ist.

**21.** Verfahren zur Herstellung einer übersättigten Lösung von Pazopanib, wobei das Verfahren umfasst:

Bilden einer wässrigen Lösung eines
Säureadditionssalzes von Pazopanib und eines modifizierten Cyclodextrins, das zur Verwendung als ophthalmische Formulierung geeignet ist; und
Einstellen des pH-Wertes dieser Lösung zwischen 3,5 und 5,7, um eine übersättigte Lösung von Pazopanib zu erhalten, wobei die Konzentration des Säureadditionssalzes von Pazopanib, das in der übersättigten Lösung aufgelöst ist, äquivalent zu etwa 10 mg/ml Pazopanib ist.

**22.** Verfahren gemäss Anspruch 21, wobei das Säureadditionssalz von Pazopanib Pazopanib-Hydrochlorid ist.

**23.** Verfahren gemäss Anspruch 21 oder 22, wobei das modifizierte Cyclodextrin aus der Gruppe bestehend aus Hydroxypropyl-β-cyclodextrin, Methyl-β-cyclodextrin, β-Cyclodextrinsulfobutylether und Kombinationen davon ausgewählt ist.

## Revendications

1. Composition pharmaceutique comprenant :

   environ 10 mg de pazopanib/ml de la composition ;
   environ 2,0 à environ 13,0 % p/p d'une cyclodextrine modifiée ;
   un agent ajustant le pH selon les besoins pour fournir un pH de 3,5 à 5,7 ;
   un agent ajustant la tonicité selon les besoins pour fournir une osmolalité de 200 à 400 mOsm ; et
   de l'eau.

2. Composition pharmaceutique selon la revendication 1, comprenant :

   environ 10 mg de pazopanib/ml de la composition ;
   environ 2,0 à environ 13,0 % p/p d'une cyclodextrine modifiée, ladite cyclodextrine modifiée étant choisie de telle manière que la cyclodextrine modifiée entraîne un $pK_a$ du pazopanib avec ladite cyclodextrine modifiée dans de l'eau inférieur au $pK_a$ du pazopanib seul dans de l'eau ;
   un agent ajustant le pH selon les besoins pour fournir un pH de 3,5 à 5,7 ;
   un agent ajustant la tonicité selon les besoins pour fournir une osmolalité de 200 à 400 mOsm ; et
   de l'eau ;
   la composition étant stable pendant au moins 2 mois.

3. Composition pharmaceutique selon la revendication 2, dans laquelle la cyclodextrine modifiée est choisie de telle manière que la cyclodextrine modifiée entraîne un $pK_a$ du pazopanib avec ladite cyclodextrine modifiée dans de l'eau au moins inférieur de 0,4 au $pK_a$ du pazopanib seul dans de l'eau dans une solution de 10 mg de pazopanib/ml d'eau.

4. Composition pharmaceutique selon l'une quelconque des revendications 2 ou 3, dans laquelle la cyclodextrine modifiée est choisie de telle manière que la cyclodextrine modifiée entraîne un $pK_a$ du pazopanib avec ladite cyclodextrine modifiée dans de l'eau au moins inférieur de 0,8 au $pK_a$ du pazopanib seul dans de l'eau.

5. Composition pharmaceutique selon la revendication 1, comprenant :

   environ 10 mg de pazopanib/ml de la composition ;
   environ 2,0 à environ 13,0 % p/p d'une cyclodextrine modifiée ; et
   un agent ajustant le pH selon les besoins pour fournir un pH de 3,5 à 5,7 ;
   un agent ajustant la tonicité selon les besoins pour fournir une osmolalité de 200 à 400 mOsm ; et
   de l'eau ;
   la composition ayant une valeur $U_{CD}$ située dans la plage de 0,0002 à 0,6 à une température de 25 °C, et la composition étant stable pendant au moins 2 mois.

6. Composition pharmaceutique selon la revendication 1, comprenant :

   environ 10 mg de pazopanib/ml de la composition ;
   environ 2,0 à environ 13,0 % p/p d'une cyclodextrine modifiée ; et
   un agent ajustant le pH selon les besoins pour fournir un pH de 3,5 à 5,7 ;
   un agent ajustant la tonicité selon les besoins pour fournir une osmolalité de 200 à 400 mOsm ; et
   de l'eau ;
   la composition étant une solution aqueuse sursaturée de pazopanib, et la composition étant stable pendant au moins 2 mois.

7. Composition pharmaceutique selon l'une quelconque des revendications précédentes, la composition ayant un pH d'environ 4 à environ 4,5.

8. Composition pharmaceutique selon les revendications 1 à 6, l'osmolalité de la composition étant d'environ 270 à environ 330 mOsm.

9. Composition pharmaceutique selon les revendications 1 à 6, dans laquelle la cyclodextrine modifiée est choisie dans le groupe constitué d'hydroxypropyl-β-cyclodextrine, méthyl-β-cyclodextrine, sulfobutyléther de β-cyclodextrine

et leurs combinaisons.

**10.** Composition pharmaceutique selon la revendication 9, dans laquelle la cyclodextrine modifiée est le sulfobutyléther de β-cyclodextrine.

**11.** Composition pharmaceutique selon les revendications 9 et 10, dans laquelle la quantité de la cyclodextrine modifiée se situe dans la plage d'environ 6,0 % à environ 10,0 % p/p.

**12.** Composition pharmaceutique selon l'une quelconque des revendications précédentes, dans laquelle la cyclodextrine modifiée est appropriée pour une administration à l'oeil d'un être humain.

**13.** Composition pharmaceutique selon l'une quelconque des revendications précédentes, la composition étant une formulation de gouttes pour les yeux appropriée pour une administration à un être humain.

**14.** Composition pharmaceutique selon l'une quelconque des revendications précédentes, la composition étant stable pendant au moins 6 mois.

**15.** Composition pharmaceutique selon l'une quelconque des revendications précédentes, la composition étant stable pendant au moins 12 mois.

**16.** Composition pharmaceutique selon l'une quelconque des revendications précédentes, comprenant en outre un agent tampon.

**17.** Composition pharmaceutique selon la revendication 15, dans laquelle ledit agent tampon est un agent tampon phosphate.

**18.** Composition pharmaceutique selon l'une quelconque des revendications précédentes, dans laquelle l'agent ajustant le pH est choisi dans le groupe constitué d'hydroxyde de sodium, d'acide chlorhydrique et de leurs combinaisons.

**19.** Composition pharmaceutique selon la revendication 1, comprenant :

environ 10 mg de pazopanib/ml de la composition ;
environ 9,0 % de sulfobutyléther de
β-cylodextrine ;
un agent ajustant le pH selon les besoins pour fournir un pH de 3,5 à 5,7 ;
un agent ajustant la tonicité selon les besoins pour fournir une osmolalité de 200 à 400 mOsm ; et
de l'eau.

**20.** Composition pharmaceutique selon la revendication 19, la composition étant une formulation de gouttes pour les yeux appropriée pour une administration à un être humain.

**21.** Procédé de préparation d'une solution sursaturée de pazopanib, ledit procédé comprenant :

la formation d'une solution aqueuse d'un sel d'addition d'acide de pazopanib et d'une cyclodextrine modifiée appropriée pour une utilisation dans une formulation ophtalmique ; et
l'ajustement du pH de ladite solution entre 3,5 et 5,7 pour obtenir une solution sursaturée de pazopanib, où la concentration du sel d'addition d'acide du pazopanib solubilisé dans la solution sursaturée est équivalente à environ 10 mg/ml de pazopanib.

**22.** Procédé selon la revendication 21, dans lequel le sel d'addition d'acide du pazopanib est le chlorhydrate de pazopanib.

**23.** Procédé selon les revendications 21 ou 22, dans lequel la cyclodextrine modifiée est choisie dans le groupe constitué d'hydroxypropyl-β-cyclodextrine, méthyl-β-cyclodextrine, sulfobutyléther de β-cylodextrine et leurs combinaisons.

Figure 1

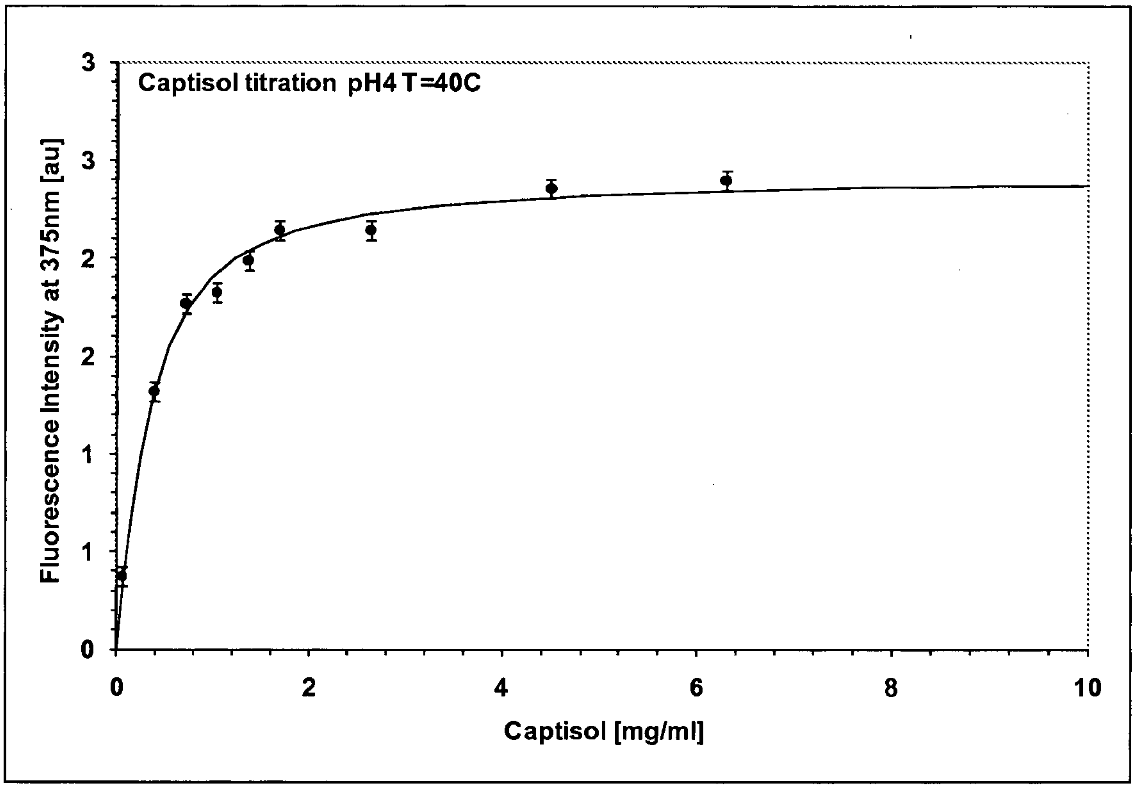
Captisol titration pH4 T=40C
Fluorescence Intensity at 375nm [au]
3
3
2
2
1
1
0
0
2
4
6
8
10
Captisol [mg/ml]

Figure 2

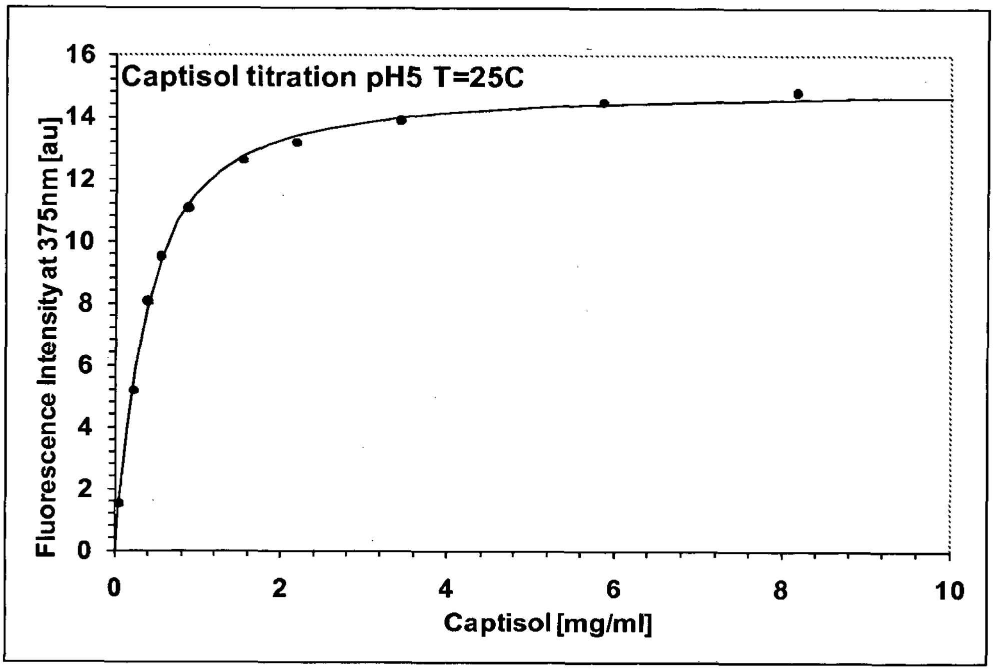
Captisol titration pH5 T=25C
Fluorescence Intensity at 375nm [au]
0
2
4
6
8
10
12
14
16
0
2
4
6
8
10
Captisol [mg/ml]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007064752 A
- US 0149367 W
- WO 02059110 A
- US 0319211 W
- WO 03106416 A

### Non-patent literature cited in the description


- **V.M. RAO ; V.J. STELLA.** When Can Cyclodextrins Be Considered for Solubilization Purposes?. *J. Pharm. Sci.,* 2003, vol. 92 (5
- A Multi-Targeted Receptor Tyrosine Kinase Inhibitor for the Treatment of Neovascular AMD: Preclinical Support of Clinical Development of Pazopanib Eye Drops. *Association for Research in Vision and Ophthalmology (ARVO),* 06 May 2009
- **RAO, V.M. ; STELLA, V.J.** *J Pharm Sci,* 2003, vol. 92, 5-927
- Binding Constants. **KENNETH A. CONNORS.** Binding Constants. Wiley-Interscience, April 1987
- **RAO, V.M. ; STELLA, V.J.** *J Pharm Sci,* May 2003, vol. 92 (5), 927
- Critical compilation of Ionisation Constants. **PRANKERD, R.J.** Profiles of drug substances, expipients and related methodology. Academic Press-Elsevier, 2007, vol. 33
- **ALBERT AA ; SERJEANT EP.** The Determination of Ionisation Constants. Chapman and Hall, 1984